# EUROPEAN PATENT APPLICATION

(11) **EP 3 795 593 A1**
(43) Date of publication of application: **24.03.2021**
(21) Application number: 20196820.3
(22) Date of filing: 18.09.2020
(51) Int. Cl.: C07K 16/28, A61K 48/00, A61K 39/395, A61P 35/00

(54) **COMBINATION OF IMMUNE-MODULATORS WITH TUMOR SPECIFIC NEOANTIGENS FOR USE IN PREVENTION AND TREATMENT OF TUMORS**

(30) Priority: 19.09.2019 IT 201900016718
(71) Applicant: Takis S.r.l., 00128 Rome (RM) (IT)
(72) Inventor: PALOMBO, Fabio, 00185 ROMA (RM) (IT); CILIBERTO, Gennaro, 00199 ROMA (RM) (IT); ROSCILLI, Giuseppe, 00172 ROMA (RM) (IT); MARRA, Emanuele, 00071 POMEZIA (RM) (IT); CONFORTI, Antonella, 00136 ROMA (RM) (IT); AURISICCHIO, Luigi, 00041 Albano Laziale (RM) (IT)
(74) Representative: Gitto, Serena

(57) **Abstract**

The present invention relates to a personalized vaccine with antitumor activity against neoantigens, administered in combination with immune-modulator agents, in particular with an anti-CTLA-4 antibody, to treat a neoplasia or prevent the relapse of a neoplasia.

SEQUENCE LISTING

<110>  TAKIS SRL

<120>  Combination of immune-modulators with tumor specific neoantigens
       for use in prevention and treatment of tumors

<130>  BE42810

<150>  IT102019000016718
<151>  2019-09-19

<160>  62

<170>  PatentIn version 3.5

<210>  1
<211>  28
<212>  PRT
<213>  Artificial Sequence

<220>
<223>  MC38 colon tumor neoantigen

<400>  1

Gly Ile Pro Val His Leu Glu Leu Ala Ser Met Thr Asn Met Glu Leu
1               5                   10                  15

Met Ser Ser Ile Val His Gln Gln Val Phe Pro Thr
            20                  25

<210>  2
<211>  28
<212>  PRT
<213>  Artificial Sequence

<220>
<223>  MC38 colon tumor neoantigen

<400>  2

Ser Leu Val Ile Ser Ala Ser Ile Ile Val Phe Asn Leu Leu Glu Leu
1               5                   10                  15

Glu Gly Asp Tyr Arg Asp Asp His Ile Phe Ser Leu
            20                  25

<210>  3
<211>  28
<212>  PRT
<213>  Artificial Sequence

<220>
<223>  MC38 colon tumor neoantigen

<400>  3

Gly Arg Val Leu Glu Leu Phe Arg Ala Ala Gln Leu Ala Asn Asp Val

1               5                   10                  15

Val Leu Gln Ile Met Glu Leu Cys Gly Ala Thr Arg
            20                  25

<210>  4
<211>  28
<212>  PRT
<213>  Artificial Sequence

<220>
<223>  MC38 colon tumor neoantigen

<400>  4

Leu Leu Arg Leu Thr Lys Gly Arg Phe Ala Leu Met Asn Leu Lys Ala
1               5                   10                  15

Leu Asp Val Phe Gly Thr Gly Ala Ser Arg Glu Phe
            20                  25

<210>  5
<211>  28
<212>  PRT
<213>  Artificial Sequence

<220>
<223>  MC38 colon tumor neoantigen

<400>  5

Gly Glu Lys Arg Ser Ile Ser Ala Ile Arg Ser Tyr Gln Tyr Val Met
1               5                   10                  15

Lys Ile Cys Ala Ala His Leu Pro Thr Glu Ser Glu
            20                  25

<210>  6
<211>  28
<212>  PRT
<213>  Artificial Sequence

<220>
<223>  MC38 colon tumor neoantigen

<400>  6

Leu Ser Ser Cys Leu Ser Asn Phe His Phe Met Cys Ala Leu Ala Ser
1               5                   10                  15

Tyr Cys Ile Phe Gln Asp Asp Lys Lys Val Phe Cys
            20                  25

<210>  7
<211>  28

<212>  PRT
<213>  Artificial Sequence

<220>
<223>  MC38 colon tumor neoantigen

<400>  7

Thr Glu Leu Arg Met Thr Arg Ala Ile Gln Pro Gln Ile Asn Ala Phe
1               5                   10                  15

Leu Gln Gly Phe His Met Phe Ile Pro Pro Ser Leu
            20                  25

<210>  8
<211>  28
<212>  PRT
<213>  Artificial Sequence

<220>
<223>  MC38 colon tumor neoantigen

<400>  8

Gly His Gly His Trp Val Asn Thr Met Ala Leu Ser Thr Tyr Tyr Ala
1               5                   10                  15

Leu Arg Thr Gly Ala Phe Glu Pro Ala Glu Ala Thr
            20                  25

<210>  9
<211>  687
<212>  DNA
<213>  Artificial Sequence

<220>
<223>  nucleotide sequence encoding a protein comprising SEQ ID NO:1-SEQ
       ID NO:8

<400>  9
ttaattaagg gcatccccgt gcacctggag ctggccagca tgaccaacat ggagctgatg      60

agcagcatcg tgcaccagca ggtgttcccc accagcctgg tgatcagcgc cagcatcatc     120

gtgttcaacc tgctggagct ggagggcgac tacagggacg accacatctt cagcctgggc     180

agggtgctgg agctgttcag ggccgcccag ctggccaacg acgtggtgct gcagatcatg     240

gagctgtgcg gcgccaccag gctgctgagg ctgaccaagg gcaggttcgc cctgatgaac     300

ctgaaggccc tggacgtgtt cggcaccggc gccagcaggg agttcggcga gaagaggagc     360

atcagcgcca tcaggagcta ccagtacgtg atgaagatct gcgccgccca cctgcccacc     420

gagagcgagc tgagcagctg cctgagcaac ttccacttca tgtgcgccct ggccagctac     480

tgcatcttcc aggacgacaa gaaggtgttc tgcaccgagc tgaggatgac cagggccatc     540

cagccccaga tcaacgcctt cctgcagggc ttccacatgt tcatcccccc cagcctgggc     600

cacggccact gggtgaacac catggccctg agcacctact acgccctgag gaccggcgcc 660

ttcgagcccg ccgaggccac ctaatga 687

<210> 10
<211> 224
<212> PRT
<213> Artificial Sequence

<220>
<223> protein encoded by SEQ ID NO:9

<400> 10

Gly Ile Pro Val His Leu Glu Leu Ala Ser Met Thr Asn Met Glu Leu
1               5                   10                  15

Met Ser Ser Ile Val His Gln Gln Val Phe Pro Thr Ser Leu Val Ile
            20                  25                  30

Ser Ala Ser Ile Ile Val Phe Asn Leu Leu Glu Leu Glu Gly Asp Tyr
        35                  40                  45

Arg Asp Asp His Ile Phe Ser Leu Gly Arg Val Leu Glu Leu Phe Arg
    50                  55                  60

Ala Ala Gln Leu Ala Asn Asp Val Val Leu Gln Ile Met Glu Leu Cys
65                  70                  75                  80

Gly Ala Thr Arg Leu Leu Arg Leu Thr Lys Gly Arg Phe Ala Leu Met
                85                  90                  95

Asn Leu Lys Ala Leu Asp Val Phe Gly Thr Gly Ala Ser Arg Glu Phe
            100                 105                 110

Gly Glu Lys Arg Ser Ile Ser Ala Ile Arg Ser Tyr Gln Tyr Val Met
        115                 120                 125

Lys Ile Cys Ala Ala His Leu Pro Thr Glu Ser Glu Leu Ser Ser Cys
    130                 135                 140

Leu Ser Asn Phe His Phe Met Cys Ala Leu Ala Ser Tyr Cys Ile Phe
145                 150                 155                 160

Gln Asp Asp Lys Lys Val Phe Cys Thr Glu Leu Arg Met Thr Arg Ala
                165                 170                 175

Ile Gln Pro Gln Ile Asn Ala Phe Leu Gln Gly Phe His Met Phe Ile
            180                 185                 190

Pro Pro Ser Leu Gly His Gly His Trp Val Asn Thr Met Ala Leu Ser
        195                 200                 205

Thr Tyr Tyr Ala Leu Arg Thr Gly Ala Phe Glu Pro Ala Glu Ala Thr
    210                 215                 220

<210>  11
<211>  27
<212>  PRT
<213>  Artificial Sequence

<220>
<223>  CT26 tumor neoantigen

<400>  11

Tyr Arg Gly Ala Asn Leu His Leu Glu Glu Thr Leu Ala Gly Phe Trp
1               5                   10                  15

Ala Arg Leu Leu Glu Arg Leu Phe Lys Gln Leu
            20                  25

<210>  12
<211>  27
<212>  PRT
<213>  Artificial Sequence

<220>
<223>  CT26 tumor neoantigen

<400>  12

Gln Ala Ile Val Arg Gly Cys Ser Met Pro Gly Pro Trp Arg Ser Gly
1               5                   10                  15

Arg Leu Leu Val Ser Arg Arg Trp Ser Val Glu
            20                  25

<210>  13
<211>  27
<212>  PRT
<213>  Artificial Sequence

<220>
<223>  CT26 tumor neoantigen

<400>  13

Asp Lys Pro Leu Arg Arg Asn Asn Ser Tyr Thr Ser Tyr Ile Met Ala
1               5                   10                  15

Ile Cys Gly Met Pro Leu Asp Ser Phe Arg Ala
            20                  25

<210>  14
<211>  27
<212>  PRT
<213>  Artificial Sequence

<220>
<223>  CT26 tumor neoantigen

<400>  14

Gly Tyr Ile Ser Arg Val Thr Ala Gly Lys Asp Ser Tyr Ile Ala Leu
1               5                   10                  15


Val Asp Lys Asn Ile Met Gly Tyr Ile Ala Ser
            20                  25


<210>  15
<211>  27
<212>  PRT
<213>  Artificial Sequence

<220>
<223>  CT26 tumor neoantigen

<400>  15

Trp Lys Gly Gly Pro Val Lys Ile Asp Pro Leu Ala Leu Met Gln Ala
1               5                   10                  15


Ile Glu Arg Tyr Leu Val Val Arg Gly Tyr Gly
            20                  25


<210>  16
<211>  25
<212>  PRT
<213>  Artificial Sequence

<220>
<223>  CT26 tumor neoantigen

<400>  16

Lys Pro Leu Arg Arg Asn Asn Ser Tyr Thr Ser Tyr Ile Met Ala Ile
1               5                   10                  15


Cys Gly Met Pro Leu Asp Ser Phe Arg
            20                  25


<210>  17
<211>  27
<212>  PRT
<213>  Artificial Sequence

<220>
<223>  CT26 tumor neoantigen

<400>  17

Met Lys Ala Phe Ile Phe Lys Tyr Ser Ala Lys Thr Gly Phe Thr Lys
1               5                   10                  15

Leu Ile Asp Ala Ser Arg Val Ser Glu Thr Glu
            20                  25

<210>  18
<211>  25
<212>  PRT
<213>  Artificial Sequence

<220>
<223>  CT26 tumor neoantigen

<400>  18

His Ser Gly Gln Asn His Leu Lys Glu Met Ala Ile Ser Val Leu Glu
1               5                   10                  15

Ala Arg Ala Cys Ala Ala Ala Gly Gln
            20                  25

<210>  19
<211>  27
<212>  PRT
<213>  Artificial Sequence

<220>
<223>  CT26 tumor neoantigen

<400>  19

Asp Gly Gln Leu Glu Leu Leu Ala Gln Gly Ala Leu Asp Asn Ala Leu
1               5                   10                  15

Ser Ser Met Gly Ala Leu His Ala Leu Arg Pro
            20                  25

<210>  20
<211>  26
<212>  PRT
<213>  Artificial Sequence

<220>
<223>  CT26 tumor neoantigen

<400>  20

Ser Trp Ile His Cys Trp Lys Tyr Leu Ser Val Gln Ser Ser Gln Leu
1               5                   10                  15

Phe Arg Gly Ser Ser Leu Leu Phe Arg Arg
            20                  25

<210>  21
<211>  25
<212>  PRT
<213>  Artificial Sequence

<220>
<223>  CT26 tumor neoantigen

<400>  21

Val Ile Gln Thr Ser Lys Tyr Tyr Met Arg Asp Val Ile Ala Ile Glu
1               5                   10                  15


Ser Ala Trp Leu Leu Glu Leu Ala Pro
            20                  25



<210>  22
<211>  27
<212>  PRT
<213>  Artificial Sequence

<220>
<223>  CT26 tumor neoantigen

<400>  22

Val Thr Ser Ile Pro Ser Val Ser Asn Ala Leu Asn Trp Lys Glu Phe
1               5                   10                  15


Ser Phe Ile Gln Ser Thr Leu Gly Tyr Val Ala
            20                  25



<210>  23
<211>  27
<212>  PRT
<213>  Artificial Sequence

<220>
<223>  CT26 tumor neoantigen

<400>  23

Glu Gly Asp Pro Cys Leu Arg Ser Ser Asp Cys Ile Asp Glu Phe Cys
1               5                   10                  15


Cys Ala Arg His Phe Trp Thr Lys Ile Cys Lys
            20                  25



<210>  24
<211>  25
<212>  PRT
<213>  Artificial Sequence

<220>
<223>  CT26 tumor neoantigen

<400>  24

Leu Arg Thr Ala Ala Tyr Val Asn Ala Ile Glu Lys Ile Phe Lys Val
1               5                   10                  15

Tyr Asn Glu Ala Gly Val Thr Phe Thr
            20                  25

<210>  25
<211>  27
<212>  PRT
<213>  Artificial Sequence

<220>
<223>  CT26 tumor neoantigen

<400>  25

Ala Gly Thr Gln Cys Glu Tyr Trp Ala Ser Arg Ala Leu Asp Ser Glu
1               5                   10                  15

His Ser Ile Gly Ser Met Ile Gln Leu Pro Gln
            20                  25

<210>  26
<211>  27
<212>  PRT
<213>  Artificial Sequence

<220>
<223>  CT26 tumor neoantigen

<400>  26

Glu His Ile His Arg Ala Gly Gly Leu Phe Val Ala Asp Ala Ile Gln
1               5                   10                  15

Val Gly Phe Gly Arg Ile Gly Lys His Phe Trp
            20                  25

<210>  27
<211>  27
<212>  PRT
<213>  Artificial Sequence

<220>
<223>  CT26 tumor neoantigen

<400>  27

Ser His Asp Ser Arg Lys Ser Thr Ser Phe Met Ser Val Asn Pro Ser
1               5                   10                  15

Lys Glu Ile Lys Ile Val Ser Ala Val Arg Arg
            20                  25

<210>  28
<211>  27
<212>  PRT
<213>  Artificial Sequence

<220>
<223>  CT26 tumor neoantigen

<400>  28

Leu Arg Ser Ser Leu Val Asn Asn Arg Thr Gln Ala Lys Ile Ala Glu
1               5                   10                  15

Glu Leu Gly Met Gln Glu Tyr Ala Ile Thr Asn
            20                  25

<210>  29
<211>  27
<212>  PRT
<213>  Artificial Sequence

<220>
<223>  CT26 tumor neoantigen

<400>  29

Ala Ala Tyr Lys Gly His His Tyr Pro Gly Pro Gly Asn Tyr Phe Trp
1               5                   10                  15

Lys Cys Leu Phe Met Ser Gly Leu Ser Glu Val
            20                  25

<210>  30
<211>  25
<212>  PRT
<213>  Artificial Sequence

<220>
<223>  CT26 tumor neoantigen

<400>  30

Ile Leu Pro Gln Ala Pro Ser Gly Pro Ser Tyr Ala Thr Tyr Leu Gln
1               5                   10                  15

Pro Ala Gln Ala Gln Met Leu Thr Pro
            20                  25

<210>  31
<211>  529
<212>  PRT
<213>  Artificial Sequence

<220>
<223>  Protein encoded by SEQ ID NO:32

<400>  31

Tyr Arg Gly Ala Asn Leu His Leu Glu Glu Thr Leu Ala Gly Phe Trp
1               5                   10                  15

Ala Arg Leu Leu Glu Arg Leu Phe Lys Gln Leu Gln Ala Ile Val Arg
            20                  25                  30

Gly Cys Ser Met Pro Gly Pro Trp Arg Ser Gly Arg Leu Leu Val Ser
        35                  40                  45

Arg Arg Trp Ser Val Glu Asp Lys Pro Leu Arg Arg Asn Asn Ser Tyr
    50                  55                  60

Thr Ser Tyr Ile Met Ala Ile Cys Gly Met Pro Leu Asp Ser Phe Arg
65                  70                  75                  80

Ala Gly Tyr Ile Ser Arg Val Thr Ala Gly Lys Asp Ser Tyr Ile Ala
                85                  90                  95

Leu Val Asp Lys Asn Ile Met Gly Tyr Ile Ala Ser Trp Lys Gly Gly
            100                 105                 110

Pro Val Lys Ile Asp Pro Leu Ala Leu Met Gln Ala Ile Glu Arg Tyr
        115                 120                 125

Leu Val Val Arg Gly Tyr Gly Lys Pro Leu Arg Arg Asn Asn Ser Tyr
    130                 135                 140

Thr Ser Tyr Ile Met Ala Ile Cys Gly Met Pro Leu Asp Ser Phe Arg
145                 150                 155                 160

Met Lys Ala Phe Ile Phe Lys Tyr Ser Ala Lys Thr Gly Phe Thr Lys
                165                 170                 175

Leu Ile Asp Ala Ser Arg Val Ser Glu Thr Glu His Ser Gly Gln Asn
            180                 185                 190

His Leu Lys Glu Met Ala Ile Ser Val Leu Glu Ala Arg Ala Cys Ala
        195                 200                 205

Ala Ala Gly Gln Asp Gly Gln Leu Glu Leu Leu Ala Gln Gly Ala Leu
    210                 215                 220

Asp Asn Ala Leu Ser Ser Met Gly Ala Leu His Ala Leu Arg Pro Ser
225                 230                 235                 240

Trp Ile His Cys Trp Lys Tyr Leu Ser Val Gln Ser Ser Gln Leu Phe
                245                 250                 255

Arg Gly Ser Ser Leu Leu Phe Arg Arg Val Ile Gln Thr Ser Lys Tyr
            260                 265                 270

Tyr Met Arg Asp Val Ile Ala Ile Glu Ser Ala Trp Leu Leu Glu Leu
        275                 280                 285

Ala Pro Val Thr Ser Ile Pro Ser Val Ser Asn Ala Leu Asn Trp Lys
    290                 295                 300

Glu Phe Ser Phe Ile Gln Ser Thr Leu Gly Tyr Val Ala Glu Gly Asp
305                 310                 315                 320

Pro Cys Leu Arg Ser Ser Asp Cys Ile Asp Glu Phe Cys Cys Ala Arg
                325                 330                 335

His Phe Trp Thr Lys Ile Cys Lys Leu Arg Thr Ala Ala Tyr Val Asn
            340                 345                 350

Ala Ile Glu Lys Ile Phe Lys Val Tyr Asn Glu Ala Gly Val Thr Phe
        355                 360                 365

Thr Ala Gly Thr Gln Cys Glu Tyr Trp Ala Ser Arg Ala Leu Asp Ser
    370                 375                 380

Glu His Ser Ile Gly Ser Met Ile Gln Leu Pro Gln Glu His Ile His
385                 390                 395                 400

Arg Ala Gly Gly Leu Phe Val Ala Asp Ala Ile Gln Val Gly Phe Gly
                405                 410                 415

Arg Ile Gly Lys His Phe Trp Ser His Asp Ser Arg Lys Ser Thr Ser
            420                 425                 430

Phe Met Ser Val Asn Pro Ser Lys Glu Ile Lys Ile Val Ser Ala Val
        435                 440                 445

Arg Arg Leu Arg Ser Ser Leu Val Asn Asn Arg Thr Gln Ala Lys Ile
    450                 455                 460

Ala Glu Glu Leu Gly Met Gln Glu Tyr Ala Ile Thr Asn Ala Ala Tyr
465                 470                 475                 480

Lys Gly His His Tyr Pro Gly Pro Gly Asn Tyr Phe Trp Lys Cys Leu
                485                 490                 495

Phe Met Ser Gly Leu Ser Glu Val Ile Leu Pro Gln Ala Pro Ser Gly

            500                 505                 510

Pro Ser Tyr Ala Thr Tyr Leu Gln Pro Ala Gln Ala Gln Met Leu Thr
        515                 520                 525

Pro

<210> 32
<211> 1587
<212> DNA
<213> Artificial Sequence

<220>
<223> nucleotide sequence encoding a protein comprising SEQ ID
NO:11-SEQ ID NO:30

<400> 32
tacaggggcg ccaacctgca cctggaggag accctggccg gcttctgggc caggctgctg 60

gagaggctgt tcaagcagct gcaggccatc gtgaggggct gcagcatgcc cggcccctgg 120

aggagcggca ggctgctggt gagcaggagg tggagcgtgg aggacaagcc cctgaggagg 180

aacaacagct acaccagcta catcatggcc atctgcggca tgcccctgga cagcttcagg 240

gccggctaca tcagcagggt gaccgccggc aaggacagct acatcgccct ggtggacaag 300

aacatcatgg gctacatcgc cagctggaag ggcggccccg tgaagatcga ccccctggcc 360

ctgatgcagg ccatcgagag gtacctggtg gtgaggggct acggcaagcc cctgaggagg 420

aacaacagct acaccagcta catcatggcc atctgcggca tgcccctgga cagcttcagg 480

atgaaggcct tcatcttcaa gtacagcgcc aagaccggct tcaccaagct gatcgacgcc 540

agcagggtga gcgagaccga gcacagcggc cagaaccacc tgaaggagat ggccatcagc 600

gtgctggagg ccagggcctg cgccgccgcc ggccaggacg gccagctgga gctgctggcc 660

cagggcgccc tggacaacgc cctgagcagc atgggcgccc tgcacgccct gaggcccagc 720

tggatccact gctggaagta cctgagcgtg cagagcagcc agctgttcag gggcagcagc 780

ctgctgttca ggagggtgat ccagaccagc aagtactaca tgagggacgt gatcgccatc 840

gagagcgcct ggctgctgga gctggccccc gtgaccagca tccccagcgt gagcaacgcc 900

ctgaactgga aggagttcag cttcatccag agcaccctgg gctacgtggc cgagggcgac 960

ccctgcctga ggagcagcga ctgcatcgac gagttctgct gcgccaggca cttctggacc 1020

aagatctgca agctgaggac cgccgcctac gtgaacgcca tcgagaagat cttcaaggtg 1080

tacaacgagg ccggcgtgac cttcaccgcc ggcacccagt gcgagtactg ggccagcagg 1140

gccctggaca gcgagcacag catcggcagc atgatccagc tgccccagga gcacatccac 1200

agggccggcg gcctgttcgt ggccgacgcc atccaggtgg gcttcggcag gatcggcaag 1260

cacttctgga gccacgacag caggaagagc accagcttca tgagcgtgaa ccccagcaag 1320

gagatcaaga tcgtgagcgc cgtgaggagg ctgaggagca gcctggtgaa caacaggacc 1380

caggccaaga tcgccgagga gctgggcatg caggagtacg ccatcaccaa cgccgcctac 1440

aagggccacc actaccccgg ccccggcaac tacttctgga agtgcctgtt catgagcggc 1500

ctgagcgagg tgatcctgcc ccaggccccc agcggcccca gctacgccac ctacctgcag 1560

cccgcccagg cccagatgct gaccccc 1587

<210> 33
<211> 15
<212> PRT
<213> Artificial Sequence

<220>
<223> 15mer mutated of SEQ ID NO:18

<400> 33

Gln Asn His Leu Lys Glu Met Ala Ile Ser Val Leu Glu Ala Arg
1               5                   10                  15

<210> 34
<211> 15
<212> PRT
<213> Artificial Sequence

<220>
<223> Wild type sequence of SEQ ID NO:33

<400> 34

Gln Asn His Leu Lys Glu Met Ala Ile Pro Val Leu Glu Ala Arg
1               5                   10                  15

<210> 35
<211> 15
<212> PRT
<213> Artificial Sequence

<220>
<223>
15mer mutated of SEQ ID NO:18

<400> 35

Lys Glu Met Ala Ile Ser Val Leu Glu Ala Arg Ala Cys Ala Ala
1               5                   10                  15

<210> 36
<211> 15
<212> PRT
<213> Artificial Sequence

<220>
<223> Wild type sequence of SEQ ID NO:35

<400>  36

Lys Glu Met Ala Ile Pro Val Leu Glu Ala Arg Ala Cys Ala Ala
1               5                   10                  15


<210>  37
<211>  15
<212>  PRT
<213>  Artificial Sequence

<220>
<223>  15mer mutated of SEQ ID NO:21

<400>  37

Gln Thr Ser Lys Tyr Tyr Met Arg Asp Val Ile Ala Ile Glu Ser
1               5                   10                  15


<210>  38
<211>  15
<212>  PRT
<213>  Artificial Sequence

<220>
<223>  Wild type sequence of SEQ ID NO:37

<400>  38

Gln Thr Ser Lys Tyr Tyr Met Arg Asp Val Thr Ala Ile Glu Ser
1               5                   10                  15


<210>  39
<211>  15
<212>  PRT
<213>  Artificial Sequence

<220>
<223>  15mer mutated of SEQ ID NO:21

<400>  39

Tyr Tyr Met Arg Asp Val Ile Ala Ile Glu Ser Ala Trp Leu Leu
1               5                   10                  15


<210>  40
<211>  15
<212>  PRT
<213>  Artificial Sequence

<220>
<223>  Wild type sequence of SEQ ID NO:39

<400>  40

Tyr Tyr Met Arg Asp Val Thr Ala Ile Glu Ser Ala Trp Leu Leu
1               5                   10                  15

<210>  41
<211>  15
<212>  PRT
<213>  Artificial Sequence

<220>
<223>  15mer mutated of SEQ ID NO:30

<400>  41

Leu Pro Gln Ala Pro Ser Gly Pro Ser Tyr Ala Thr Tyr Leu Gln
1               5                   10                  15


<210>  42
<211>  15
<212>  PRT
<213>  Artificial Sequence

<220>
<223>  Wild type sequence of SEQ ID NO:41

<400>  42

Leu Pro Gln Ala Pro Ser Gly Pro Ser Tyr Ala Ile Tyr Leu Gln
1               5                   10                  15


<210>  43
<211>  15
<212>  PRT
<213>  Artificial Sequence

<220>
<223>  15mer mutated of SEQ ID NO:30

<400>  43

Pro Ser Gly Pro Ser Tyr Ala Thr Tyr Leu Gln Pro Ala Gln Ala
1               5                   10                  15


<210>  44
<211>  15
<212>  PRT
<213>  Artificial Sequence

<220>
<223>  Wild type sequence of SEQ ID NO:43

<400>  44

Pro Ser Gly Pro Ser Tyr Ala Ile Tyr Leu Gln Pro Ala Gln Ala
1               5                   10                  15


<210>  45
<211>  15
<212>  PRT
<213>  Artificial Sequence

<220>
<223>  15mer mutated of SEQ ID NO:30

<400>  45

Ser Tyr Ala Thr Tyr Leu Gln Pro Ala Gln Ala Gln Met Leu Thr
1               5                   10                  15


<210>  46
<211>  15
<212>  PRT
<213>  Artificial Sequence

<220>
<223>  Wild type sequence of SEQ ID NO:45

<400>  46

Ser Tyr Ala Ile Tyr Leu Gln Pro Ala Gln Ala Gln Met Leu Thr
1               5                   10                  15


<210>  47
<211>  15
<212>  PRT
<213>  Artificial Sequence

<220>
<223>  15mer mutated of SEQ ID NO:20

<400>  47

His Cys Trp Lys Tyr Leu Ser Val Gln Ser Ser Gln Leu Phe Arg
1               5                   10                  15


<210>  48
<211>  15
<212>  PRT
<213>  Artificial Sequence

<220>
<223>  Wild type sequence of SEQ ID NO:47

<400>  48

His Cys Trp Lys Tyr Leu Ser Val Gln Ser Gly Gln Leu Phe Arg
1               5                   10                  15


<210>  49
<211>  15
<212>  PRT
<213>  Artificial Sequence

<220>
<223>  15mer mutated of SEQ ID NO:20

<400>  49

Tyr Leu Ser Val Gln Ser Ser Gln Leu Phe Arg Gly Ser Ser Leu
1               5                   10                  15

<210>  50
<211>  15
<212>  PRT
<213>  Artificial Sequence

<220>
<223>  Wild type sequence of SEQ ID NO:49

<400>  50

Tyr Leu Ser Val Gln Ser Gly Gln Leu Phe Arg Gly Ser Ser Leu
1               5                   10                  15


<210>  51
<211>  15
<212>  PRT
<213>  Artificial Sequence

<220>
<223>  15mer mutated of SEQ ID NO:6

<400>  51

Pro Leu Arg Arg Asn Asn Ser Tyr Thr Ser Tyr Ile Met Ala Ile
1               5                   10                  15


<210>  52
<211>  15
<212>  PRT
<213>  Artificial Sequence

<220>
<223>  Wild type sequence of SEQ ID NO:51

<400>  52

Pro Leu Arg Arg Asn Asn Ser Tyr Thr Ser Tyr Thr Met Ala Ile
1               5                   10                  15


<210>  53
<211>  15
<212>  PRT
<213>  Artificial Sequence

<220>
<223>  15mer mutated of SEQ ID NO:6

<400>  53

Asn Asn Ser Tyr Thr Ser Tyr Ile Met Ala Ile Cys Gly Met Pro
1               5                   10                  15


<210>  54
<211>  15
<212>  PRT
<213>  Artificial Sequence

<220>
<223>  Wild type sequence of SEQ ID NO:53

<400>  54

Asn Asn Ser Tyr Thr Ser Tyr Thr Met Ala Ile Cys Gly Met Pro
1               5                   10                  15


<210>  55
<211>  15
<212>  PRT
<213>  Artificial Sequence

<220>
<223>  15mer mutated of SEQ ID NO:6

<400>  55

Thr Ser Tyr Ile Met Ala Ile Cys Gly Met Pro Leu Asp Ser Phe
1               5                   10                  15


<210>  56
<211>  15
<212>  PRT
<213>  Artificial Sequence

<220>
<223>  Wild type sequence of SEQ ID NO:55

<400>  56

Thr Ser Tyr Thr Met Ala Ile Cys Gly Met Pro Leu Asp Ser Phe
1               5                   10                  15


<210>  57
<211>  15
<212>  PRT
<213>  Artificial Sequence

<220>
<223>  15mer mutated of SEQ ID NO:12

<400>  57

Gly Cys Ser Met Pro Gly Pro Trp Arg Ser Gly Arg Leu Leu Val
1               5                   10                  15


<210>  58
<211>  15
<212>  PRT
<213>  Artificial Sequence

<220>
<223>  Wild type sequence of SEQ ID NO:57

<400>  58

Gly Cys Ser Met Pro Gly Pro Trp Gly Ser Gly Arg Leu Leu Val
1               5                   10                  15

<210>  59
<211>  15
<212>  PRT
<213>  Artificial Sequence

<220>
<223>  15mer mutated of SEQ ID NO:12

<400>  59

Pro Gly Pro Trp Arg Ser Gly Arg Leu Leu Val Ser Arg Arg Trp
1               5                   10                  15


<210>  60
<211>  15
<212>  PRT
<213>  Artificial Sequence

<220>
<223>  Wild type sequence of SEQ ID NO:59

<400>  60

Pro Gly Pro Trp Gly Ser Gly Arg Leu Leu Val Ser Arg Arg Trp
1               5                   10                  15


<210>  61
<211>  15
<212>  PRT
<213>  Artificial Sequence

<220>
<223>  15mer mutated of SEQ ID NO:26

<400>  61

His Ile His Arg Ala Gly Gly Leu Phe Val Ala Asp Ala Ile Gln
1               5                   10                  15


<210>  62
<211>  15
<212>  PRT
<213>  Artificial Sequence

<220>
<223>  Wild type sequence of SEQ ID NO:61

<400>  62

His Ile His Arg Ala Gly Gly Leu Phe Val Ala Asp Glu Ile Gln
1               5                   10                  15

## Description

The present invention relates to the combination of modulators with tumor-specific neoantigens for use in the prevention and treatment of tumors.

In particular, the invention relates to a personalized vaccine with antitumor activity against neoantigens, administered in combination with immune modulator agents, in particular with an anti-CTLA-4 antibody, to treat a neoplasia or prevent the relapse of a neoplasia.

It is well known that the effectiveness of oncological immunotherapy has opened new treatment perspectives for many tumors. The main actors in this revolution are immune checkpoint inhibitors (ICIs),¹ that is, therapeutic antibodies against the brakes on the immune response, in particular PD1 and CTLA-4. The latter are two receptors differently expressed by tumor cells and by lymphocyte subpopulations that block T-lymphocytes against the tumor.

However, ICI-based treatments, though approved as first-line treatments for a vast number of clinical indications, are not always effective and have major side effects, especially in treatments combined with other therapies. Lying at the basis of these undesirable effects is a general activation of the immune system, which attacks normal tissues in a nonspecific manner.

In the light of the foregoing, it appears evident that there is a need to provide new therapies for cancer prevention and treatment which overcome the disadvantages of the known therapies.

It has recently been understood that the mechanism of effectiveness of ICIs is mediated by cytotoxic T responses directed against the mutated regions of tumor proteins. These mutations, if expressed on a protein level, can be presented to the immune system and thus be subject to the cytotoxic T response. These targets are known by a name of neoantigens.²

The possibility of characterizing the repertoire of neoantigens of a tumor is the result of the new nucleic acid sequencing technology known as next generation sequencing (NGS), which has led to the decoding of tumor mutations, their frequency and the specific allele expression in tens and tens of thousands of tumors, from the most common, such as lung cancer, to the rarest, like melanoma.

The association between the effectiveness of immunotherapy and the expressed mutations of the tumor is the precondition for exploring the idea of a personalized vaccine that can teach the immune system of the host to recognize tumors in a specific and lasting manner (induction of immunological memory). This concept finds scientific support in the many preclinical studies on tumor-specific vaccines (neoantigen cancer vaccine - NCV) and in several clinical studies.³⁻⁷ Clinical studies of 2017⁴ had already highlighted the need to include a number of potentially immunogenic neoantigens greater than 5, as initially indicated in studies on mice²¹ and in the first clinical study with dendritic cells.³ The difference is not given by the number of neoantigens inserted in the vaccine, which can also be greater than 30 as in the case of adenoviruses,⁸ but rather by the number of actually effective CD8-specific neoantigens, as previously demonstrated.⁹

Fitting into this context is the solution according to the present invention, which aims to provide new methods and instruments for cancer immunotherapy via the administration of a combination of a personalized vaccine, based on specific neoantigens of the individual tumor, employed alone or in association with tumor antigens overexpressed by the tumor (CEA, Her2, NY-ESO1) and of one or more anti-CTLA-4 antibodies, optionally in association with other immune modulator agents.

According to the present invention, in fact, it was surprisingly found that the combination of the neoantigen-based vaccine with the anti-CTLA-4 immune modulator is capable of significantly inhibiting tumor growth, whereas combinations of the same vaccine with other immune modulators did not yield satisfactory results.

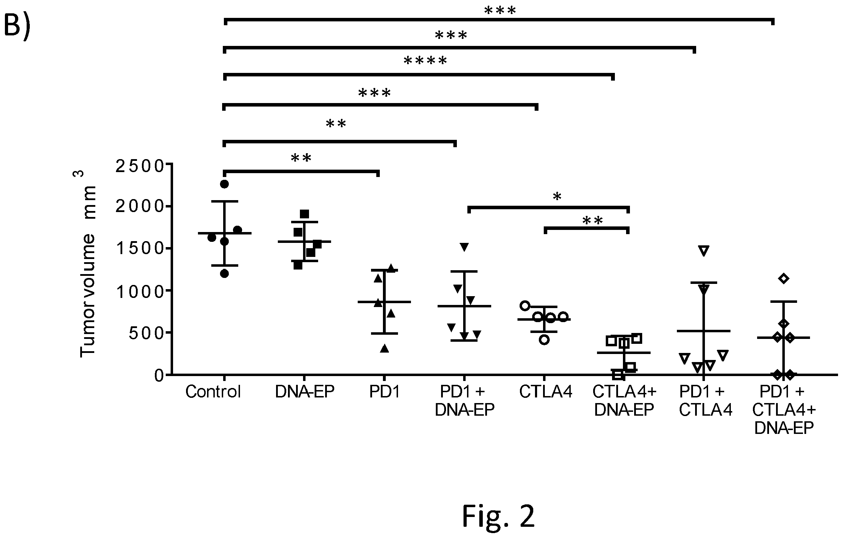

In particular, in example 1 given further below, use was made of several neoantigens known in the literature. The nucleotide sequences encoding the neoantigens were inserted into a plasmid DNA vector in the form of linker-free 28mers and administered by electroporation (DNA-EP). It was sought to understand which neoantigens were immunogenic and whether they were effective in therapeutic tumor protection. In this context, it was sought to understand whether the combination with ICIs was necessary, in particular with anti-PD1, anti-CTLA-4 or both. As noted above, it was observed that only the combination of the vaccine with anti-CTLA-4 significantly inhibits tumor growth. This observation was confirmed following the administration of the vaccine and anti-CTLA-4 in association also with anti-PD1, whereas significant results were not obtained through the co-treatment of the vaccine in combination with anti-PD1 in the absence of anti-CTLA-4 (Fig. 2B). With the aim of understanding the antitumor mechanisms involved, in example 1 the immune responses against the neoantigens induced by the pTKopt vaccine were analyzed. A strong effector CD8 response (detected by IFN-γ ELISPOT) was observed only when the vaccine was administered with anti-CTLA-4 or with both anti-CTLA-4 and anti-PD1. This result is surprising, as many studies have demonstrated that the combination with anti-PD1 on its own or with both anti-PD1 and anti-CTLA-4 is necessary to render the therapeutic vaccine effective. However, no one had ever first observed an antitumor impact mediated by a strong effector response (IFNγ⁺) against tumor neoantigens only with anti-CTLA-4. In example 1 it is shown that the co-treatment of the vaccine (comprising only neoantigens) with anti-PD1 has no impact on the immune response induced by the vaccine.

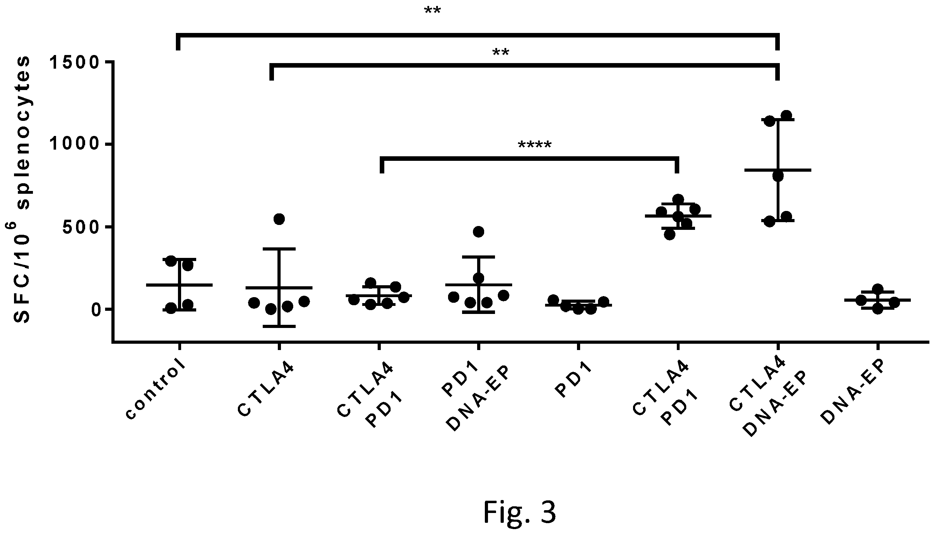

In the light of the evidence in example 1 with MC38, it was further assessed whether an increase in the number of neoantigens expressed by the vaccine was capable of further improving the therapeutic effects. In particular, it was assessed whether the effectiveness of the vaccine depended on the number of neoantigens expressed by the tumor and therefore whether an increase in effectiveness was observed when the number of neoantigens produced by the tumor increases. From an analysis of databases such as the TCGA it emerged that a significant number of a large variety of tumors, including, for example, melanoma or lung cancer, express twenty or more neoantigens with high affinity. The results of example 2 show that the combination of a DNA vaccine comprising 20 neoantigens with anti-CTLA-4 induces a complete tumor regression in a CT26 model. The results were better compared to those obtained with a DNA vaccine that uses a smaller number of neoantigens, like the one used in example 1. These results indicate that the effectiveness of the vaccine is better in tumors expressing a number of neoantigens of at least twenty. The effectiveness of the vaccination system and of the immunological correlates (such as, for example, the results of the analysis of the specific responses for the neoantigens, Fig. 3) according to the present invention indicates that this system can be advantageously applied in clinical practice in the context of therapy with anti-CTLA-4. To this end, a process for producing the vaccine on the scale necessary for clinical application was also developed. Personalized vaccination requires a production that is as rapid as possible, especially in therapeutic contexts. The present invention proposes a process for producing a personalized vaccine, in particular DNA vaccines to be administered by EP, in the amount necessary for clinical therapy, and which enables the product to be obtained in about a week, in particular 6 days. This result is possible thanks to the use of single-use systems and to the optimization of the scale of production, as described in example 2.

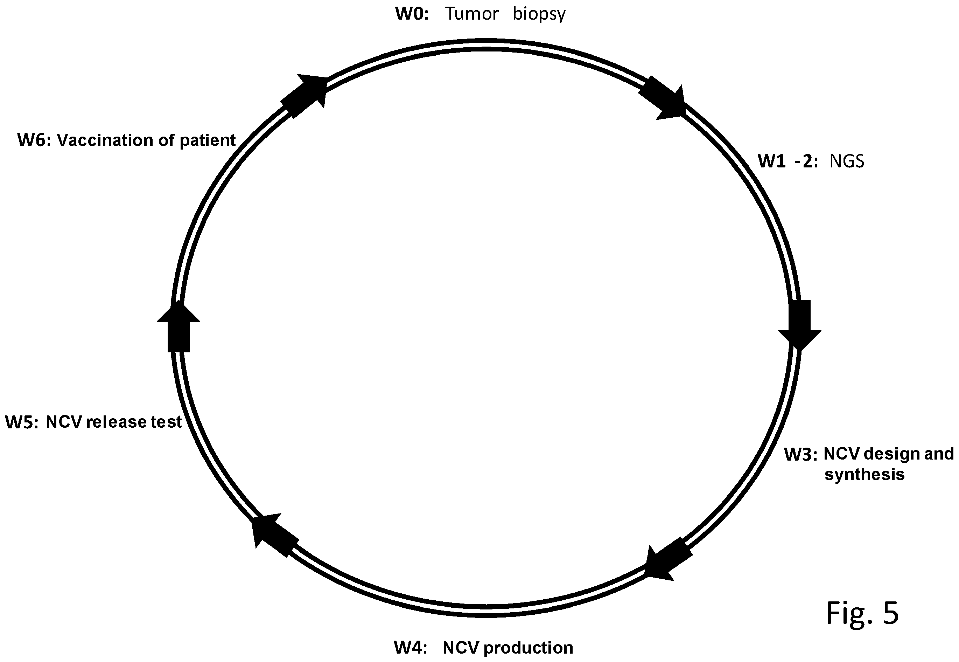

According to the present invention, the complete personalized vaccination process, starting from the biopsy and ending with administration of the vaccine, can thus have an overall duration of about six weeks (Fig.5). The target patients identified include patients affected by melanoma, lung cancer and kidney cancer, for which therapy with ICIs is already approved, and patients affected by other histological types of tumor, as described below.

These and other results are obtained according to the present invention, by proposing the combination of one or more tumor neoantigens (at least eight and preferably at least twenty), used as a vaccine, with one or more modulators of the immune response, preferably anti-CTLA-4 antibodies, for use in the prevention and treatment of tumors, wherein the vaccine is administered by electroporation under particular conditions. Therefore, the present invention provides a method for treating or preventing a tumor in a patient who needs treatment, the method comprising the administration of:
- a personalized vaccine in the form of a vector or plasmid comprising nucleotide sequences encoding neoantigens, for example administered into muscle by means of the electroporation technique; and
- an immune checkpoint inhibitor, preferably an anti-CTLA-4. According to one embodiment of the present invention the immune checkpoint inhibitor is other than anti-PD1.

The administration can be sequential (first one and then the other component) or concomitant. The number of administrations can be one or two, with two-weekly, monthly, six-monthly and yearly frequencies. The administration of the ICI can also be repeated with a similar frequency.

According to the present invention, neoantigens means tumor antigens codified by nucleotide sequences of the tumor cells, said sequences comprising somatic mutations. The mutations can be identified by comparing the tumor DNA with the DNA of healthy tissue. The expression of the neoantigens can be subsequently confirmed by analyzing the sequence of the tumor RNA. These expressed mutations can then be analyzed by means of a prediction algorithm which evaluates the probability of them being present on the surface of the tumor and recognized by a cytotoxic T-cell.

According to the present invention, the individual neoantigens are preferably selected independently from point mutations, deletions or insertions, and splice-site mutations.

The T-cell-mediated immune response recognizes the mutated peptides (neoepitopes) as non-self and thus activates a cytotoxic response to kill the cells expressing them. The term non-self is used in a manner similar to that concerning the responses against viral epitopes which evoke, precisely, a response that eliminates the cell containing the virus and thus the infection. In the majority of the cases studied, the neoantigens induce a specific cytotoxic T response for the mutation which does not cross-react with the natural sequence, thus revealing not only specificity for the tumor, but also the absence of toxicity against normal tissues that express the natural unmutated allele.

A further important aspect of the neoantigen-based vaccine according to the present invention is the possibility of striking a vast number of specific targets of the tumor, the neoantigens precisely. In particular, this multi-target approach is independent of the biological function of the neoantigens and can be redesigned by following the mutational evolution of the tumor over time, i.e., it is possible to insert into the vaccine new neoantigens identified in a tumor relapse. In this regard, with the aim of preventing a possible tumor relapse, it is possible, according to the present invention, to vaccinate the patient against targets (neoantigens) with a low allele frequency, which could prevail in a relapse.

The vaccination vectors according to the present invention can be administered by EP. The principles of electroporation are very simple. The lipid membrane of a cell can be considered as a dielectric element interposed between the extracellular environment and the cytoplasmatic environment, which are both conductive. An electric field applied to a cell induces a transmembrane potential: when the dielectric potential of the membrane is exceeded, transient pores appear in the membrane, a process called electroporation^{10,11}. If the electric field is maintained for a sufficiently long time, the membrane becomes permeable (electropermeabilization) because the transient pores are stabilized and become large enough to allow charged macromolecules, such as DNA, to access the cytoplasm. The cells remain in a porated state for a limited period of time and close rapidly after the electric treatment ends. The duration of the electric pulse must be sufficiently short to avoid irreversible damage to the cell membrane and cell death. The transmembrane potential increases linearly with the intensity of the applied electric field, but above a certain threshold (generally 0.5-2 V) it decreases, indicating that the conductivity of the membrane increases due to the formation of hydrophilic pores.^{12,13} As the molecules of a nucleic acid such as DNA or RNA are too large to penetrate through hydrophilic pores simply by diffusion, an electrophoretic field must be maintained for a sufficient time in order to allow the polyanions to move and enter the cell. The DNA must be in proximity to the cell membrane before the electric field is applied.¹⁰ The DNA molecules pass through the pores of the membrane by means of an electrodiffusive process. It is postulated that the progression of the DNA towards the nucleus takes place through a combination of classic electrophoresis and passive diffusion according to a concentration gradient. Therefore, the pulses must be optimized so as to obtain the best combination of cell permeabilization followed by the desired electrophoretic effect. In general, the pulse parameters are arbitrarily divided into short high-voltage pulses, greater than 400V/cm with a duration in the gamma range of µsec and low-voltage pulses, less than 400 V/cm with a duration in the interval of msec. An efficient gene transfer has been demonstrated using either only a sequence of high-voltage pulses or only low-voltage pulses. However, in theory the most effective strategy seems to be a combination of short initial high-voltage pulses followed by a sequence of longer lasting low-voltage pulses. Protein expression in muscle is usually improved 100-1000 times after electroporation of DNA (DNA_EP) compared to the injection of naked DNA, thanks above all to greater cellular absorption.¹⁴⁻¹⁶ Various devices for DNA-EP exist. The most advanced technologies are the ones being developed by Inovio Pharmaceuticals (http://www.inovio.com), Ichor Medical Systems (http://www.ichorms.com) and IGEA (http://www.igeamedical.com ).

Skeletal muscle is the most frequent target organ for DNA-EP. Skeletal muscles are easily accessible beneath the skin and are made up of post-mitotic cells capable of long-term expression of the transgene after transfection.¹⁷ Furthermore, the tissue damage is rapidly repaired, without signs of muscle degeneration.¹⁸ Muscle DNA-EP is an invasive procedure that requires needles for injection of the nucleic acid, followed by the electric discharge. In small animals this is achieved with flat electrodes positioned on the skin around the injected volume, whereas in larger species, including humans, it requires an array of needles inserted into the tissue. In addition to administration into muscle, electroporation is used for the administration of DNA into the skin using much shorter needles and under slightly different electric conditions. In clinical studies, these procedures have demonstrated to be well tolerated and not to cause severe pain¹⁹.

The method of administration of the vaccine is not limited solely to plasmid DNA, as it can be given in the form of peptides or viral vectors in a prime/boost sequence. The plasmid vaccine with EP can be used for maintaining the immune response over time after viral vaccines, which are neutralized by the immune response against the viral vector already after the first administration, i.e., adenovirus-derived vectors.

One advantage of the present invention is the absence of a complex formulation as in the case of lipoparticles with peptides or RNA, as well of nanoparticles of other nature loaded with the vaccine. The plasmid DNA vaccine is formulated in a standard buffer and administered by EP.

The method of vaccination based on an expression plasmid and delivered by EP is known in the art. In fact, the construction of a plasmid for vaccination that has the tissue-type plasminogen activator (TPA) as a leader sequence followed by an antigen is already described in a previous patent (WO2001045748A1) and is by now part of standard laboratory techniques.

It is therefore a specific object of the present invention a combination of a first component A comprising:
a modulator of the immune response, preferably an anti-CTLA-4 antibody, or
a polynucleotide encoding said modulator, or
an expression vector or plasmid which comprises the polynucleotide encoding said modulator with
a second component B comprising:
   a polynucleotide encoding one or more neoantigens, or
   an expression vector or plasmid comprising said polynucleotide encoding one or more neoantigens,
   said combination being for simultaneous, separate or sequential use in the prevention and treatment of the tumor.

Preferably, said combination does not comprise anti-PD1 antibodies.

Moreover, the combination according to the present invention does not comprise a further antitumor vaccine other than component B of the present invention.

The combination according to the present invention can thus comprise one or more modulators of the immune response or polynucleotides encoding said modulators. Furthermore, the combination can comprise a single polynucleotide encoding one or more neoantigens or more than one polynucleotide encoding different neoantigens. The polynucleotide sequences encoding the neoantigens comprise a leader sequence at the 5' end. The leader sequence has the function of transporting the neoantigens outside the cell of the organism transfected with the vector or plasmid, for example by electroporation. An example of a leader sequence is TPA.

The combination can also comprise a single polynucleotide comprising both a nucleotide sequence encoding one or more modulators and a nucleotide sequence encoding one or more neoantigens. The sequences can also comprise linkers.

According to one embodiment, the combination of the invention can comprise a polynucleotide encoding a fusion protein of said one or more neoantigens fused to the profilin-like protein of Toxoplasma gondii (PFTG).

According to the present invention, the vector or polynucleotide is a DNA vaccine.

According to the present invention, "simultaneous use" means the administration of the components of the combination in a single, identical pharmaceutical form, for example when all the components of the combination are polynucleotides or vectors.

"Separate use" means the administration, at the same time, of the components of the combination in distinct pharmaceutical forms, for example an injectable form and a form suitable for electroporation to be administered simultaneously.

"Sequential use" means the sequential administration of the components of the combination, each in a distinct pharmaceutical form. Therefore, according to the present invention the modulators of the immune response, preferably the anti-CTLA-4 antibody, can be administered before said vaccine, after said vaccine or simultaneously with said vaccine. Preferably, the vaccine can be administered, for example by electroporation, before the administration of the modulator of the immune response, for example in an injectable pharmaceutical form.

According to the present invention, a T-type cell immune response against at least 4 tumor-specific neoantigens is preferably induced in the patient.

The neoantigens according to the present invention can be administered to a mammal, in particular a human being, a dog, a cat, a horse, a cow, a mouse or a rat.

A vector according to the present invention comprises nucleotide sequences that can be under the transcriptional control of a promoter.

According to one aspect, the present invention also relates to a collection of expression vectors according to the present invention, the vectors preferably consisting in a plasmid, an RNA, an RNA formulated with an adjuvant, a replicating RNA, or a replicating RNA formulated with an adjuvant.

The expression vector is preferably a plasmid that is administered by electroporation, preferably into the muscle, into the dermis, into the tumor or subcutaneously. The electroporation treatment can comprise the use of deep or surface electrodes, flat electrodes and/or needles. The conditions of electroporation, for example in the muscle, are preferably the following low voltage electric conditions: 8 pulses of 20 msec each of 110V, 8Hz, with an interval of 120 msec between each of them.

The expression vector which comprises the polynucleotide of the invention, operatively bound to a promoter or to a regulatory transcriptional element, can be selected for example from: bacterial plasmids, adenovirus, poxvirus, vaccinia virus, fowlpox, herpes virus, adeno-associated virus (AAV), alphavirus, lentivirus, lambda phage, lymphocytic choriomeningitis virus, Listeria sp and Salmonella sp.

The vaccine according to the present invention can be advantageously administered in a single site or in more sites. For example, for a vaccine comprising 40 neoantigens, 10 neoantigens can be administered into the quadriceps of the right leg, the next 10, different from the first, into the quadriceps of the left leg, the next 10 neoantigens into the deltoid of the right arm and the next 10 neoantigens into the deltoid of the left arm.

According to the present invention, said modulator of the immune response can be selected in the group consisting of anti- CD27, CD40, OX40, GITR, PD1, PD-L1, CD274, A2AR, B7-H3, B7-H4, BTLA, CTLA-4, IDO, KIR, LAG3, TIM-3, VISTA, IL-2, IL-12, IL-15 antibodies.

According to one embodiment, said polynucleotide encoding said modulator and/or said one or more neoantigens or expression vector or plasmid comprising said polynucleotide can be administered by electroporation.

Preferably, said polynucleotide encoding one or more neoantigens comprises a leader sequence, for example TPA, at the in the 5' end.

According to the present invention, said one or more neoantigens can be at least 3 neoantigens, preferably from 3 to 50 neoantigens, more preferably at least 3, 5, 8, 10, 20, 30, 40, for example 3, 5, 8, 10, 20, 30, 40, or 50 neoantigens, even more preferably at least 20, alone or in combination with known tumor antigenic epitopes, with different levels of affinity for MHC classes I and II.

According to the present invention, when said polynucleotide encodes more than one neoantigen, the neoantigens can derive either from frameshift mutations or other types of mutations, such as, for example, point mutations. In particular, when said polynucleotide encodes at least 20 neoantigens, the neoantigens are derived not only from frameshift mutations, but also from other types of mutations, such as, for example, point mutations.

Preferably, according to the present invention, the aforesaid binding affinity with MHC has values lower than 500 nM and, even more preferably, values lower than 50 nM.

As mentioned above, said one or more neoantigens can be comprised in a same polypeptide or in different polypeptides, for example at least 8 neoantigens can be comprised in one polypeptide and optionally fused to a leader sequence such as TPA.

According to the present invention, said one or more neoantigens can have a length comprised between 9 and 35 amino acids, for example a length from 9 to 29 amino acids or from 15 to 35 amino acids.

According to one embodiment of the invention, the NCV (Neoantigen Cancer Vaccine) consists of a sequence of 28 amino acids with the mutated amino acid at the center in the case of point mutations, or of the entire immunogenic sequence predicted on the basis of the HLA expressed in the patient, in the case of *frameshift* or *intron retention.*

According to one embodiment of the present invention, the combination can further comprise an immune response promoter (for example a probiotic) and/or an antineoplastic agent. The antineoplastic agent that can be present in the combination of the invention can be an anti-angiogenic agent, an alkylating agent, an antimetabolite, a natural product, a platinum coordination complex, an anthracenedione, a substituted urea, a methylhydrazine derivative, an adrenocortical inhibitor, a hormone, an antagonist, an oncogenic inhibitor, an oncosuppressor gene or protein, a therapeutic antibody and an anti-oncogene oligonucleotide.

The tumors that can be treated with the combination of the present invention can be selected in the group consisting of melanoma, lung cancer, tumors of the urinary system, such as, for example, of the kidney, urethra or bladder, tumors of the digestive system, such as, for example, of the esophagus, stomach, intestine, colon, colon/rectum, anus, liver, gall bladder, biliary ducts or pancreas, breast cancer, eye tumors, brain tumors, lymphomas, such as, for example, Hodgkin and non-Hodgkin, multiple myeloma, leukemias, such as, for example, myeloid and lymphocytic chronic leukemia, and/or tumors of the reproductive system, such as, for example, of the cervix, endometrium, ovary, prostate, testicles, penis or vulva. Preferably, the tumors that can be treated according to the present invention are tumors that express at least 20 neoantigens, such as, for example, melanoma and lung cancer.

A further object of the present invention is a kit for the prevention and treatment of tumors, said kit comprising:
a) a first component A comprising:
   a modulator of the immune response, preferably an anti-CTLA-4 antibody, or
   a polynucleotide encoding said modulator, or
   an expression vector or plasmid which comprises the polynucleotide encoding said modulator;
b) a second component B comprising:
   a polynucleotide encoding one or more neoantigens, or
   an expression vector or plasmid comprising said polynucleotide encoding one or more neoantigens;
c) a system of administration by electroporation.

The combination according to the present invention can be advantageously used in a method for preventing, treating or slowing tumors in a mammal (such as, for example, a human or an animal such as, for example, a dog), which comprises administering said combination to a mammal, for which said prevention and slowing treatment is necessary or desirable. As a consequence of the administration, an immune response is generated against said tumor, which is thus prevented, treated or slowed. Among the tumors that can be treated mention may be made of tumors of the adrenal gland, anus, auditory nerve, biliary ducts, bladder, bones, brain, breast, central nervous system, cervix, colon, ear, endometrium, esophagus, eye, palpebra, Fallopian tube, gastrointestinal tract, head-neck, heart, kidneys, larynx, liver, lung, mandible, mandibular condyle, maxilla, mouth, nasopharynx, nose, oral cavity, ovary, pancreas, parotid gland, penis, pinna, pituitary gland, prostate gland, rectum, retina, salivary glands, skin, small intestine, spine, stomach, testicle, thyroid, urethra, vagina, vestibulocochlear nerve and vulva. As a result of the administration of the combination according to the present invention immune responses of the cytotoxic T-cell type are generated, which exhibit properties of inhibiting the proliferation of tumor cells expressing the target antigen.

The DNA vaccine according to the present invention can be prepared by means of a process which comprises a) preparing a nucleotide sequence encoding an antigen sequence which consists of or comprises one or more neoantigens; b) cloning the nucleotide sequence in an expression vector or plasmid for expression in cells of a mammal; and c) amplifying the vector plasmid in a suitable bacterial microorganism, and isolating the same from the microorganism.

The method for producing the personalized neoantigen-based vaccine according to the present invention in 6 weeks comprises a first step of acquiring biopsies of the patient for molecular analysis. This first step consists in the extraction of RNA and DNA from the tumor and DNA from peripheral blood. These nucleic acids are sent to a laboratory, which within a week performs exome sequencing both of the tumor DNA and of the normal DNA and RNAseq of the tumor. These data are used to determine the somatic mutations: missense, frameshift, intron retention and exome skipping. The translocations are instead analyzed by RNAseq. The HLA of the patient is determined using the exome sequencing data. The somatic mutations are then analyzed for the expression level of the mutated allele using the RNAseq data. Only those expressed above the threshold of about 10 reads go on to the subsequent analysis for prediction of the specific HLA binding of the patient. The neoantigens with a predicted HLA binding value below 500 nM or preferably below 50 nM (detected by means of NetMHC-4 and NetMHCpan 20 software) are selected for the vaccine. The minimum number of neoantigens in order for patients to be eligible is 10, better if 20, even better if 40.

The peptide sequences can be constructed in such a way that they have the mutation at the center and 14 amino acids per side. This vaccine design makes it possible to induce not only CD8 epitopes, but also CD4, as described in clinical studies.^{3,4} The sequences are extracted from genomics data and linked to one another in such a way as to generate a polypeptide. This is reverse transcribed using codon optimization for humans. The vaccine sequence is sent to an outside provider which performs the synthesis and cloning in the pTK1A-TPA vector. The final vector, once sequenced to confirm the correctness of the synthesis, is sent to a GMP-certified manufacturer which provides for the production of 200 mg of plasmid, which, in addition to treatment of the patient, will serve for the drug release analysis.

The present invention will now be described by an illustrative but not limitative way, according to a preferred embodiment thereof, with particular reference to examples and to the figures of the appended drawings, wherein:
- **Figure 1** shows A) a scheme for neoantigen identification and generation of a vaccine, pTKopt, B) the analysis of the immune responses induced by pTKopt against the individual neoantigens 1 to 8 listed in table 1 and analyzed by ELISPOT. Spot forming colony (SFC);
- **Figure 2** shows **A)** the growth over time of the MC38 tumors under different treatment conditions and **B)** the final measurements of the tumor growth and statistical analysis (T-test). *<0.05; **<0.01; ***< 0.001; ****<0.0001;
- **Figure 3** shows the immune response analysis by ELISPOT;
- **Figure 4** shows the growth over time of the CT26 tumors under different treatment conditions;
- **Figure 5** shows a time diagram of the clinical process of development of the personalized vaccine against the tumor; and
- **Figure 6** shows the agarose gel of the DNA produced with the fermentation process. M= marker, lane 1 supercoiled DNA, lane 2 linearized DNA, lane 3 expected insert release according to the experimental design.

### EXAMPLE 1. Neoantigen identification process, development of a personalized vaccine against the neoantigens and treatment of mice with the vaccine in combination with ICI according to the present invention.

### Neoantigen identification process, development of a personalized vaccine against the neoantigens

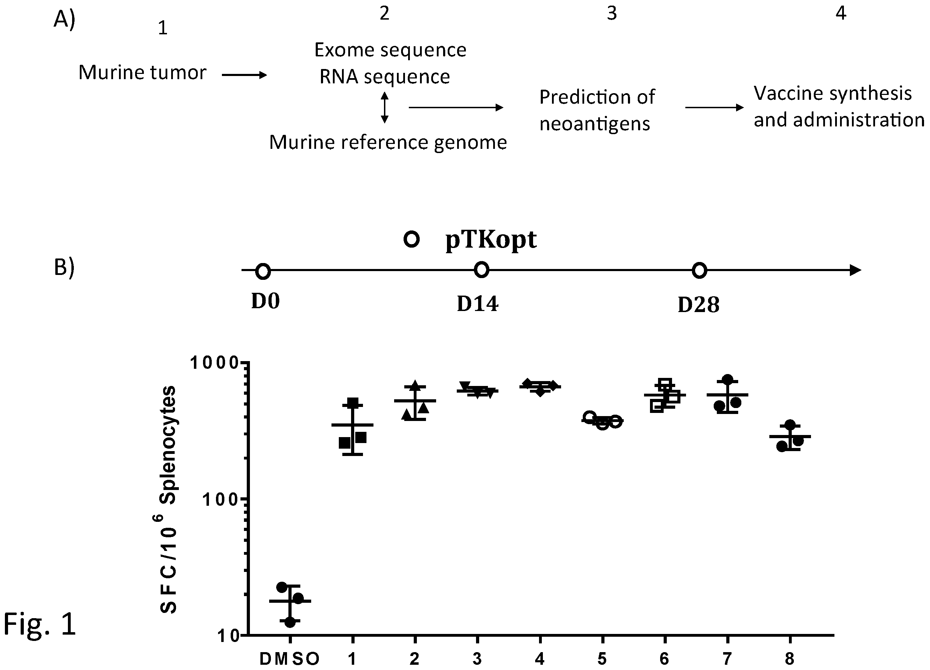

The method used to identify the neoantigens and generate the NCV is based on the following four steps (Fig.1):
1) collection of tumors and normal samples;
2) sequencing and identification of the neoantigens;
3) vaccine design and preparation;
4) administration of the vaccine by electroporation.

In this case, in the murine system, the specific point mutations of the tumor were identified by comparing the sequencing data of the exome of the MC38 (ATCC) tumor cells line with reference to the mouse genome.

In humans, by contrast, the tumor is compared with a normal tissue (e.g. PBMC).

In order to be immunogenic, the neoantigen must be expressed and visible to the immune system. Therefore, the mutations were further selected on the basis of the gene expression level measured by RNAseq (RNA sequence). Finally, the neoantigens expressed were classified on the basis of different bioinformatic conducts. The most popular methods for predicting the association with MHC are NetMHC-4 and NetMHCpan 20.

The sequences were then incorporated into the pTK1-TPA expression vector generating the pTK-opt vector and administered by electroporation.

The pTK1-TPA expression vector comprises the promoter and intron A from human cytomegalovirus (CMV), a polylinker site for cloning and bovine growth hormone (bGH) as a polyA for the transcription termination. Furthermore, the vector also comprises the leader sequence of tissue plasminogen activator (tPA).

C57/B6 mice were vaccinated with 3 vaccinations with pTKopt in the presence of EP every two weeks and the immune responses were measured in the splenocytes 1 week after the last vaccination (Fig.1B). The experiment confirmed the immunogenicity of all the known neoantigens.^{9 22} This result demonstrates that with a genetic vaccination based on DNA with EP it is possible to design a vaccine without the need to use a linker between one neoantigen and the other. Furthermore, the immunogenicity is independent not only of the length of the peptide but also of the position that the neoantigen occupies in the sequence of the polypeptide. The latter observation is evident for sequences 2,3,4,5,6,7,8, which in previous experiments reported in the literature occupied different positions in the polypeptide compared to pTKopt⁹.

### Treatment of mice with the vaccine against neoantigens of MC38 colon tumor in combination with ICIs according to the present invention

*With reference to Article 170bis of the Italian Industrial Property Code, it is hereby declared that the studies on genetically modified organisms cited below took place inside a facility with a biosafety level of BSL2, with notification ID RM*/*IC*/*Imp2*/*04*/*001, Takis s.r.l. authorized on 09*/*04*/*2015.*

With the aim of obtaining a T-cell response against the neoantigens of interest, a genetic vaccination based on DNA electroporation into skeletal muscles was adopted. This technology enables the expression of a sequence of different neoantigens appropriately engineered in order to present them to the immune system and induce an effector response against the tumor. The plasmid vector used, pTKopt, encodes 8 neoantigens expressed by MC38 colon tumor cells, whose amino acid sequences are listed below in Table 1 and which were identified as previously described.

**Table 1.**

| **TK-M-opt neoantigens** | **SEQ ID** |
|---|---|
| GIPVHLELASMTN**M**ELMSSIVHQQVFPT | SEQ ID NO:1 |
| SLVISASIIVFNL**L**ELEGDYRDDHIFSL | SEQ ID NO:2 |
| GRVLELFRAAQL**A**NDVVLQIMELCGATR | SEQ ID NO:3 |
| LLRLTKGRFALMN**L**KALDVFGTGASREF | SEQ ID NO:4 |
| GEKRSISAIRSYQ**Y**VMKICAAHLPTESE | SEQ ID NO:5 |
| LSSCLSNFHFMCA**L**ASYCIFQDDKKVFC | SEQ ID NO:6 |
| TELRMTRAIQPQI**N**AFLQGFHMFIPPSL | SEQ ID NO:7 |
| GHGHWVNTMALST**Y**YALRTGAFEPAEAT | SEQ ID NO:8 |

The mutation is expressed at the center with about 14 amino acids at the N and C terminals. The sequence of the neoantigens was cloned inframe with the TPA signal and followed by two stop codons and expressed under the control of the CMV promoter. The genetic code was optimized for expression in the mouse. The nucleotide sequence cloned in the vector is: 
TTAATTAAGGGCATCCCCGTGCACCTGGAGCTGGCCAGCATG
ACCAACATGGAGCTGATGAGCAGCATCGTGCACCAGCAGGTGTTCC
CCACCAGCCTGGTGATCAGCGCCAGCATCATCGTGTTCAACCTGCTG
GAGCTGGAGGGCGACTACAGGGACGACCACATCTTCAGCCTGGGCA
GGGTGCTGGAGCTGTTCAGGGCCGCCCAGCTGGCCAACGACGTGGT
GCTGCAGATCATGGAGCTGTGCGGCGCCACCAGGCTGCTGAGGCTG
ACCAAGGGCAGGTTCGCCCTGATGAACCTGAAGGCCCTGGACGTGT
TCGGCACCGGCGCCAGCAGGGAGTTCGGCGAGAAGAGGAGCATCA
GCGCCATCAGGAGCTACCAGTACGTGATGAAGATCTGCGCCGCCCA
CCTGCCCACCGAGAGCGAGCTGAGCAGCTGCCTGAGCAACTTCCAC
TTCATGTGCGCCCTGGCCAGCTACTGCATCTTCCAGGACGACAAGAA
GGTGTTCTGCACCGAGCTGAGGATGACCAGGGCCATCCAGCCCCAG
ATCAACGCCTTCCTGCAGGGCTTCCACATGTTCATCCCCCCCAGCCT
GGGCCACGGCCACTGGGTGAACACCATGGCCCTGAGCACCTACTAC
GCCCTGAGGACCGGCGCCTTCGAGCCCGCCGAGGCCACCTAATGA
 (SEQ ID NO: 9, cDNA of the 8 neoantigens listed in table 1).

The respective amino acid sequence inserted in pTK-M-opt aa is: 
GIPVHLELASMTNMELMSSIVHQQVFPTSLVISASIIVFNLLELEGD
YRDDHIFSLGRVLELFRAAQLANDVVLQIMELCGATRLLRLTKGRFALMN
LKALDVFGTGASREFGEKRSISAIRSYQYVMKICAAHLPTESELSSCLSNF
HFMCALASYCIFQDDKKVFCTELRMTRAIQPQINAFLQGFHMFIPPSLGH
GHWVNTMALSTYYALRTGAFEPAEAT**
 (SEQ ID NO: 10, amino acid sequence of the 8 neoantigens listed in table 1).

The mice were implanted subcutaneously with the MC38 tumor cells at a dose of 3x10⁵ and two days after subjected to treatments with: DNA-EP, anti-PD1, anti-CTLA-4, anti-PD1 and anti-CTLA-4, anti-PD1+ DNA-EP and anti-CTLA-4 + DNA-EP. The vaccination protocol consisted in an injection in the quadriceps muscle of 6-7-week-old female C57/B6 mice (Envigo, the Netherlands), the DNA was formulated in phosphate-buffered saline (PBS) at a concentration of 0.5 mg/ml. The DNA-EP was performed with an IGEA Cliniporator electroporator, using a needle electrode (electrode N-10-4B). For the muscle DNA-EP, the following low-voltage electric conditions were applied: 8 pulses of 20 msec each of 110V, 8Hz, with an interval of 120 msec between each of them. The mice were vaccinated at days 2, 6 and 14 after tumor inoculation. The treatments with the anti-PD1 antibodies (BioXcell, clone RMP114) and the anti-CTLA-4 antibody (BioxCell, clone 9D9) were injected into the peritoneum at days 3, 7, 9 and 15 at a dose of 100 µgr. in 100 µl of final PBS.

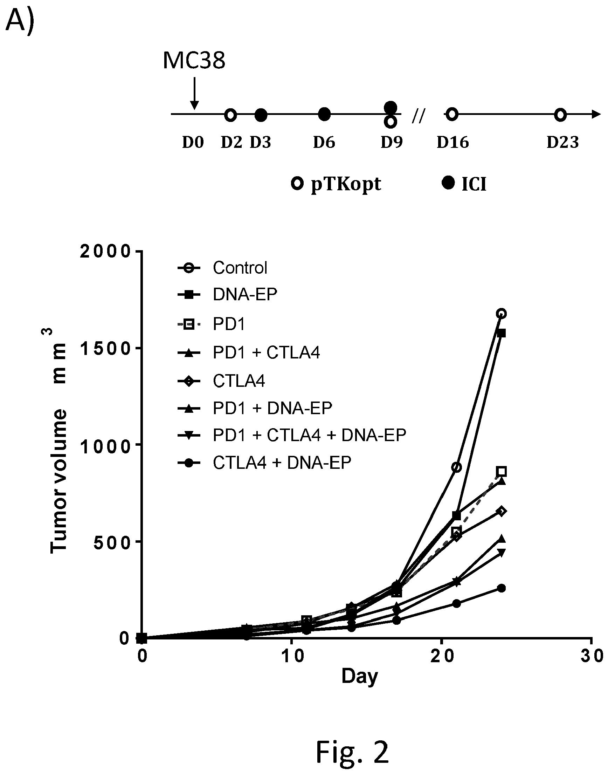

The mice treated with the pTKopt-EP vaccine and anti-CTLA-4 showed a reduced tumor growth compared to the treatment with anti-CTLA-4 alone or with the vaccine alone (Fig. 2A). As expected, the treatment with anti-PD1 reduced tumor growth in a statistically significant manner versus the control, as did the anti-CTLA-4 or the co-treatment of anti-CTLA-4 and anti-PD1 with the vaccination. Surprisingly, the vaccination improved the effect of the co-treatment in a statistically significant manner with anti-CTLA-4, but not with anti-PD1 (Fig. 2B), though both anti-PD1 and anti-CTLA-4 showed an impact on the tumor.

### The immune response specific for the neoantigens is guided by the co-treatment with the vaccine and anti-CTLA-4.

In order to understand whether the antitumor activity was mediated by an effect of the immune modulators on the immune responses induced by the vaccine we analyzed, by IFN-γ ELISPOT,⁹ the splenocytes of the mice sacrificed at the end of the experiment (Fig. 3). The immunological data reveal an unexpected mechanism. The immune modulators alone (both anti-PD1 and anti-CTLA-4), the combination thereof (anti-PD1 + anti-CTLA-4), and the vaccine in combination with anti-PD1 did not increase the specific responses against the neoantigens versus the control. The responses observed in the control are justified by the facts that three weeks after tumor inoculation, the very large tumors had induced a spontaneous response that was not observed in tumor-free mice (data not shown).

The immunological response against the neoantigens becomes statistically significant only when we combine the vaccine with anti-CTLA-4 alone or with anti-PD1. Even though not statistically significant (p=0.058), there is a trend in favor of the treatment with anti-CTLA-4 alone as compared to co-treatment with anti-PD1. This result is in line with the tumor growth data, where the best antitumor result was seen with the vaccine and anti-CTLA-4.

### EXAMPLE 2: Study on the effectiveness of the vaccine of the invention expressing 20 neoantigens in combination with CTLA-4 in CT26 tumor.

In clinical practice tumors can express a variable number of neoantigens and they can respond or not to immunotherapy. With the aim of simulating such a context, that is, a tumor other than MC38 having a high number of expressed neoantigens, CT26 were used.²¹ Therefore, in order to answer the question whether a greater number of neoantigens, i.e., 20, used in the vaccine in a different tumor had a therapeutic impact, use was made of the neoantigens of the CT26 model listed in table 2.

**Table 2**

| | |
|---|---|
| 1 | YRGANLHLEETLAGFWARLLERLFKQL (SEQ ID NO:11) |
| 2 | QAIVRGCSMPGPWRSGRLLVSRRWSVE (SEQ ID NO:12) |
| 3 | DKPLRRNNSYTSYIMAICGMPLDSFRA (SEQ ID NO:13) |
| 4 | GYISRVTAGKDSYIALVDKNIMGYIAS (SEQ ID NO:14) |
| 5 | WKGGPVKIDPLALMQAIERYLVVRGYG (SEQ ID NO:15) |
| 6 | KPLRRNNSYTSYIMAICGMPLDSFR (SEQ ID NO:16) |
| 7 | MKAFIFKYSAKTGFTKLIDASRVSETE (SEQ ID NO:17) |
| 8 | HSGQNHLKEMAISVLEARACAAAGQ (SEQ ID NO:18) |
| 9 | DGQLELLAQGALDNALSSMGALHALRP (SEQ ID NO:19) |
| 10 | SWIHCWKYLSVQSSQLFRGSSLLFRR (SEQ ID NO:20) |
| 11 | VIQTSKYYMRDVIAIESAWLLELAP (SEQ ID NO:21) |
| 12 | VTSIPSVSNALNWKEFSFIQSTLGYVA (SEQ ID NO:22) |
| 13 | EGDPCLRSSDCIDEFCCARHFWTKICK (SEQ ID NO:23) |
| 14 | LRTAAYVNAIEKIFKVYNEAGVTFT (SEQ ID NO:24) |
| 15 | AGTQCEYWASRALDSEHSIGSMIQLPQ (SEQ ID NO:25) |
| 16 | EHIHRAGGLFVADAIQVGFGRIGKHFW (SEQ ID NO:26) |
| 17 | SHDSRKSTSFMSVNPSKEIKIVSAVRR (SEQ ID NO:27) |
| 18 | LRSSLVNNRTQAKIAEELGMQEYAITN (SEQ ID NO:28) |
| 19 | AAYKGHHYPGPGNYFWKCLFMSGLSEV (SEQ ID NO:29) |
| 20 | ILPQAPSGPSYATYLQPAQAQMLTP (SEQ ID NO:30) |

### Ordering of the 20 neoantigens

In order to limit the immune responses induced by the vaccine to solely the neoantigen sequences, an algorithm was developed, Epitopes_shuffling.sh, which investigates immunogenicity at the junction sites. The code takes as input a txt file containing the list of neoantigens selected to be encoded by the vaccine, and a txt file with the specific haplotype. The concatenations are unique and randomly generated and each one is memorized in a random_permutation_n *.txt file for viewing if the downstream analysis indicates that one of the concatenations generated has succeeded. Every random concatenation of n neoantigens produces n-1 junction sites, which are extracted for the immunogenicity analysis. The code is designed to capture the junction sites of a length of 16 residues and, for each one, calculates all of the possible 9-mer peptides to be input to NetMHCpam.

The tool for predicting peptide-MHC class I binding calculates the binding affinities for the selected alleles and saves the output in an .xls file, which is further manipulated to render it suitable for the downstream analysis.

Once the predicted binding affinities are collected, a filter is applied: each random concatenation is evaluated on the basis of the binding affinity values of the deriving 9-mer peptides of the junction site. If all the binding affinity values are greater than a given personalized value (e.g. 500 nM), the concatenation has a positive outcome and could potentially be used for the construction of the plasmid.

### Construction of the CT26_20 vector

The neoantigens listed in table 2 were assembled one after the other in the following polypeptide:

YRGANLHLEETLAGFWARLLERLFKQLQAIVRGCSMPGPWRSGR
LLVSRRWSVEDKPLRRNNSYTSYIMAICGMPLDSFRAGYISRVTAGKDS
YIALVDKNIMGYIASWKGGPVKIDPLALMQAIERYLVVRGYGKPLRRNNS
YTSYIMAICGMPLDSFRMKAFIFKYSAKTGFTKLIDASRVSETEHSGQNHL
KEMAISVLEARACAAAGQDGQLELLAQGALDNALSSMGALHALRPSWIH
CWKYLSVQSSQLFRGSSLLFRRVIQTSKYYMRDVIAIESAWLLELAPVTSI
PSVSNALNWKEFSFIQSTLGYVAEGDPCLRSSDCIDEFCCARHFWTKIC
KLRTAAYVNAIEKIFKVYNEAGVTFTAGTQCEYWASRALDSEHSIGSMIQ
LPQEHIHRAGGLFVADAIQVGFGRIGKHFWSHDSRKSTSFMSVNPSKEIK
IVSAVRRLRSSLVNNRTQAKIAEELGMQEYAITNAAYKGHHYPGPGNYF
WKCLFMSGLSEVILPQAPSGPSYATYLQPAQAQMLTP (SEQ ID NO:31)

Then the polypeptide was reverse-transcribed in the sequence SEQ ID NO:32 and inserted into the pTK1-TPA vector. The pTK1-TPA expression vector comprises the promoter and intron A of human cytomegalovirus (CMV), a polylinker site for cloning, and bovine growth hormone (bGH) as a polyA for the transcription termination. Furthermore, the vector also comprises the leader sequence of tissue plasminogen activator (tPA).

SEQ ID NO:32:

TACAGGGGCGCCAACCTGCACCTGGAGGAGACCCTGGCCGGCTTCT
GGGCCAGGCTGCTGGAGAGGCTGTTCAAGCAGCTGCAGGCCATCGT
GAGGGGCTGCAGCATGCCCGGCCCCTGGAGGAGCGGCAGGCTGCT
GGTGAGCAGGAGGTGGAGCGTGGAGGACAAGCCCCTGAGGAGGAA
CAACAGCTACACCAGCTACATCATGGCCATCTGCGGCATGCCCCTGG
ACAGCTTCAGGGCCGGCTACATCAGCAGGGTGACCGCCGGCAAGGA
CAGCTACATCGCCCTGGTGGACAAGAACATCATGGGCTACATCGCCA
GCTGGAAGGGCGGCCCCGTGAAGATCGACCCCCTGGCCCTGATGCA
GGCCATCGAGAGGTACCTGGTGGTGAGGGGCTACGGCAAGCCCCTG
AGGAGGAACAACAGCTACACCAGCTACATCATGGCCATCTGCGGCAT
GCCCCTGGACAGCTTCAGGATGAAGGCCTTCATCTTCAAGTACAGCG

CCAAGACCGGCTTCACCAAGCTGATCGACGCCAGCAGGGTGAGCGA
GACCGAGCACAGCGGCCAGAACCACCTGAAGGAGATGGCCATCAGC
GTGCTGGAGGCCAGGGCCTGCGCCGCCGCCGGCCAGGACGGCCAG
CTGGAGCTGCTGGCCCAGGGCGCCCTGGACAACGCCCTGAGCAGC
ATGGGCGCCCTGCACGCCCTGAGGCCCAGCTGGATCCACTGCTGGA
AGTACCTGAGCGTGCAGAGCAGCCAGCTGTTCAGGGGCAGCAGCCT
GCTGTTCAGGAGGGTGATCCAGACCAGCAAGTACTACATGAGGGAC
GTGATCGCCATCGAGAGCGCCTGGCTGCTGGAGCTGGCCCCCGTGA
CCAGCATCCCCAGCGTGAGCAACGCCCTGAACTGGAAGGAGTTCAG
CTTCATCCAGAGCACCCTGGGCTACGTGGCCGAGGGCGACCCCTGC
CTGAGGAGCAGCGACTGCATCGACGAGTTCTGCTGCGCCAGGCACT
TCTGGACCAAGATCTGCAAGCTGAGGACCGCCGCCTACGTGAACGC
CATCGAGAAGATCTTCAAGGTGTACAACGAGGCCGGCGTGACCTTCA
CCGCCGGCACCCAGTGCGAGTACTGGGCCAGCAGGGCCCTGGACA
GCGAGCACAGCATCGGCAGCATGATCCAGCTGCCCCAGGAGCACAT
CCACAGGGCCGGCGGCCTGTTCGTGGCCGACGCCATCCAGGTGGG
CTTCGGCAGGATCGGCAAGCACTTCTGGAGCCACGACAGCAGGAAG
AGCACCAGCTTCATGAGCGTGAACCCCAGCAAGGAGATCAAGATCGT
GAGCGCCGTGAGGAGGCTGAGGAGCAGCCTGGTGAACAACAGGAC
CCAGGCCAAGATCGCCGAGGAGCTGGGCATGCAGGAGTACGCCATC
ACCAACGCCGCCTACAAGGGCCACCACTACCCCGGCCCCGGCAACT
ACTTCTGGAAGTGCCTGTTCATGAGCGGCCTGAGCGAGGTGATCCTG
CCCCAGGCCCCCAGCGGCCCCAGCTACGCCACCTACCTGCAGCCCG
CCCAGGCCCAGATGCTGACCCCC

### Immune responses induced against CT26_20 neoantigens and impact on tumor growth

The immune responses against the neoantigens were evaluated both by IFN-γ ELISPOT and by flow cytometry as reported in the literature.⁹ A group of 6 mice were vaccinated with pTK-CT26_20 every two weeks for three weeks and then sacrificed one week after the last vaccination for immunological analysis of the splenocytes stimulated with the peptides specific for the neoantigens. The data indicate a high number of IFN-g responses of both CD8+ cells and CD4+ cells determined by flow cytometry and confirmed by ELISPOT data. Table 3 shows the results of both assays.

**Table 3**

| | | | | | **ICS** | |
|---|---|---|---|---|---|---|
| **Gene** | **28mer** | **15mer mutated** | **WT** | **IFNγ ELIspot Assay (SFC 1x10⁶ cells)** | **CD4 (%)** | **CD8 (%)** |
| Aldh18a1 | HSGQNHL KEMAISV LEARACA AAGQ (SEQ ID NO:18) | QNHLKEM AISVLEAR (SEQ ID NO:33) | QNHLKEMA IPVLEAR (SEQ ID NO:34) | 141 | 0.055 | - |
| | | KEMAISVL EARACAA (SEQ ID NO:35) | KEMAIPVLE ARACAA (SEQ ID NO:36) | 124,5 | 0.08 | - |
| Dhx35 | VIQTSKYY MRDVIAI ESAWLLE LAP (SEQ ID NO:21) | QTSKYYM RDVIAIES (SEQ ID NO:37) | QTSKYYMR DVTAIES (SEQ ID NO:38) | 136 | not tested | not tested |
| | | YYMRDVIA IESAWLL (SEQ ID NO:39) | YYMRDVTAI ESAWLL (SEQ ID NO:40) | 176.5 | 0.04 | - |
| E2f8 | ILPQAPSG PSYATYLQ PAQAQM LTP (SEQ ID NO:30) | LPQAPSGP SYATYLQ (SEQ ID NO:41) | LPQAPSGPS YAIYLQ (SEQ ID NO:42) | 612 | 0.032 | - |
| | | PSGPSYAT YLQPAQA (SEQ ID NO:43) | PSGPSYAIYL QPAQA (SEQ ID NO:44) | 594 | - | 0.045 |
| | | SYATYLQP AQAQMLT (SEQ ID NO:45) | SYAIYLQPA QAQMLT (SEQ ID NO:46) | 45.5 | - | 0.44 |
| Mtch1 | SWIHCW KYLSVQSS QLFRGSSL LFRR (SEQ ID NO:20) | HCWKYLS VQSSQLFR (SEQ ID NO:47) | HCWKYLSV QSGQLFR (SEQ ID NO:48) | 822 | - | 0.24 |
| | | YLSVQSSQ LFRGSSL (SEQ ID NO:49) | YLSVQSGQL FRGSSL (SEQ ID NO:50) | 158 | not tested | not tested |
| Slc20a1 | KPLRRNN | PLRRNNSY | PLRRNNSYT | | - | 0.15 |
| | SYTSYIMA ICGMPLD SFR (SEQ ID NO:6) | TSYIMAI (SEQ ID NO:51) | SYTMAI (SEQ ID NO:52) | | | |
| | | NNSYTSYI MAICGMP (SEQ ID NO:53) | NNSYTSYT MAICGMP (SEQ ID NO:54) | 193 | 0.06 | - |
| | | TSYIMAIC GMPLDSF (SEQ ID NO:55) | TSYTMAICG MPLDSF (SEQ ID NO:56) | 110 | 0.08 | - |
| Tmem87 | QAIVRGC SMPGPW RSGRLLVS RRWSVE (SEQ ID NO:12) | GCSMPGP WRSGRLL V (SEQ ID NO:57) | GCSMPGP WGSGRLLV (SEQ ID NO:58) | 733.5 | - | 0.58 |
| | | PGPWRSG RLLVSRRW (SEQ ID NO:59) | PGPWGSGR LLVSRRW (SEQ ID NO:60) | 457 | not tested | not tested |
| Agxt2I2 | EHIHRAG GLFVADAI QVGFGRI GKHFW (SEQ ID NO:26) | HIHRAGGL FVADAIQ (SEQ ID NO:61) | HIHRAGGLF VADEIQ (SEQ ID NO:62) | 47.5 | - | - |

The mice were implanted subcutaneously with the CT26 tumor cells at a dose of 3x10⁵, they were subjected to DNA-EP treatments two days later and again treated with anti-CTLA-4 the next day. The vaccination protocol consisted in an intradermal injection of 10 ugr. of pTK-CT26_20 in 6-7-week-old female Balb/C mice (Envigo, the Netherlands); the DNA was formulated in phosphate-buffered saline (PBS) at a concentration of 0.333 mg/ml. DNA-EP was carried out with an IGEA Cliniporator electroporator, using an adjustable needle electrode (electrode N-10-4-B) under the following electric conditions: 3 pulses of 100 msec each of 26V, 8Hz, with an interval of 120 msec between each of them. The mice were vaccinated at days 2, 7 and 14 after tumor inoculation. The treatments with the anti-CTLA-4 antibody (BioxCell, clone 9D9) were performed in the peritoneum at days 3, 8 and 15 at a dose of 200 µgr. in 100 µl of final PBS.

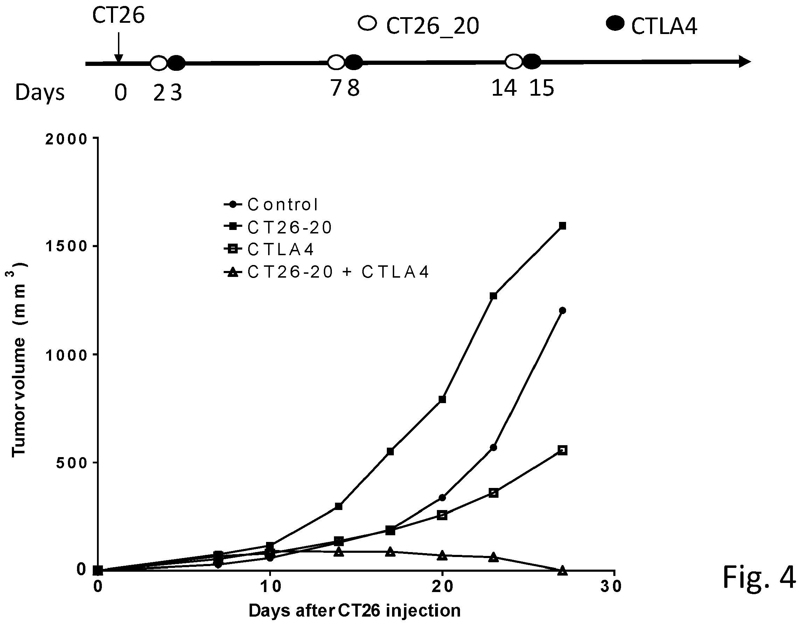

The mice treated with the pTK-CT26_20 vaccine and anti-CTLA-4 showed a complete regression of tumor growth compared to the treatment with anti-CTLA-4 alone or with the vaccine alone (Fig. 4). As expected, the treatment with anti-CTLA-4 reduced tumor growth in a statistically significant manner versus the control. Surprisingly, the vaccination improved the effect of anti-CTLA-4 in a statistically significant manner, inducing complete tumor regression.

### EXAMPLE 3. Therapeutic protocol and production of the vaccine according to the present invention in six weeks.

The method for producing the personalized neoantigen-based vaccine in 6 weeks is described in Fig. 5:
the first step is to acquire biopsies of the patient for molecular analysis. This first step consists in the extraction of RNA and DNA from the tumor and DNA from peripheral blood. These nucleic acids are sent to an outside laboratory, which within a week performs exome sequencing both of the tumor DNA and of the normal DNA and RNAseq of the tumor. These data are used to determine the somatic mutations: missense, frameshift, intron retention and exome skipping. The translocations are instead analyzed by RNAseq. The HLA of the patient is determined using the exome sequencing data. The somatic mutations are then analyzed for the expression level of the mutated allele using the RNAseq data. Only those expressed above the threshold of about 10 reads passes on to the subsequent analysis for prediction of the specific HLA binding of the patient. The neoantigens with a predicted HLA binding value below 500 nM or preferably below of 50 nM (NetMHC-4 and NetMHCpan, ref. 20) are selected for the vaccine. The minimum number of neoantigens in order for patients to be eligible is 10, better if 20, even better if 40.

The peptide sequences are constructed in such a way that they have the mutation at the center and 14 amino acids per side. This vaccine design makes it possible to induce not only CD8 epitopes, but also CD4, as described in clinical studies.^{3,4}

The sequences are extracted from genomics data and linked to one another in the ordering generated by Epitopes_shuffling.sh (see example above) in such a way as to generate a polypeptide. This is reverse transcribed using codon optimization for humans. The sequence of the vaccine is sent to an outside provider which performs the synthesis and cloning in the pTK1A-TPA vector. The final vector, once sequenced to confirm the correctness of the synthesis, is sent to a GMP-certified manufacturer which provides for the production of 200 mg of plasmid, which, in addition to treatment of the patient, serve for the drug release analysis.

An innovative aspect of the production of the plasmid-based vaccine regards the scale of production and the time necessary, which must be as short as possible. A process was developed for the production of 200 mg of plasmid, with the characteristics required by the pharmacopeia, in a week. In particular, pTK-20neo, a vaccine containing 20 neoantigens derived from a primary line of melanoma of a patient (*the primary line was drawn after the patient's informed consent was obtained),* was produced according to the following scheme:
Day 1. Transformation of pTK-20neo in the *E. Coli* Stellar bacterial strain which was selected because of the high level of plasmid production.
Day 2. Pre-inoculation of 10 colonies in 10 ml of medium (Luria broth, LB) up to an OD of 0.4 and creation of stabs in glycerol (-80 °C).
Day 3. Inoculation of 500 mL of Luria-Bertani with one of the frozen stabs and culturing for about 6 hours in a flask before inoculating the fermenter (Bioflo, Eppendorf) containing 5 L of proprietary medium. During fermentation in the Bioflow 320 (Eppendorf), the pH was maintained constant at 7.2, the temperature at 37°, the % of O₂ above 40% and stirring at between 400 and 600 rpm (rotations per minute).
Day 4. After 16 hours of fermentation, the bacteria were centrifugated and resuspended in 2 L of buffer (50 mM Tris/HCI pH 8.0 and 10 mM EDTA), lysed with 2 L of lysis buffer (0.2N NaOH in 1% SDS) and incubated for 5 minutes, then buffered with 7M of C₂H7NO₂ in 1M of CH₃COOK and incubated at 4°C and O/N at 4°C.
Day 5. The sample was first filtered with a filter with a depth of 0.45 µm and then over a 0.22 µm filter; the permeate was diluted with water until obtaining a conductivity of 80-85 mS/cm. The first chromatography step provided for an ion exchange and elution in 200 ml of buffer containing 1.6 M NaCl, 10 mM Tris/HCI pH 8.0 and 0.1 mM EDTA. The second step for removing the endotoxins provided for a hydrophobic column. The DNA was equilibrated in 1.6 M (NH4)2SO4 and the eluate was concentrated/dialyzed by tangential ultrafiltration.

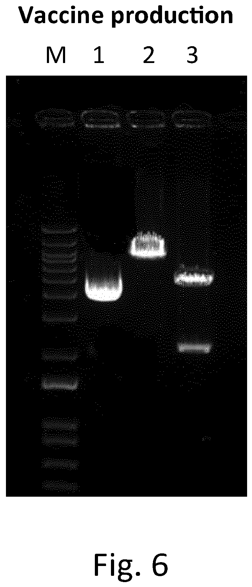

The result of the process is shown in Fig.6, where it can be seen that 213 mg. of pTK-20neo were produced, with a percentage of supercoiled DNA greater than 95% (lane 1). The digestion of the insert identifies the expected band of about 1500 nucleotides (lane 3).

The process described is capable of producing the amount of DNA necessary to treat a patient and perform the required release tests. The production time is about 6 days.

Once the release test has been performed, the vaccine is sent to the hospital, which administers the vaccine with a Cliniporator®. One example of treatment: 5 mg of vaccine formulated in 1 ml of PBS is injected into the deltoid of the patient and subjected to EP with the Cliniporator® using an N-10-4-B or a variable geometry electrode. For the DNA-EP in the muscle, the following low-voltage (LV) electric conditions are applied: 8 pulses of 20 msec each of 110V, 8Hz, interval of 120msec between each of them.

The patient is subjected to a vaccination and treatment with anti-CTLA-4 every 3 weeks as a standard treatment for melanoma.

The present invention has been described for non-limiting illustrative purposes, according to its preferred embodiments, but it is to be considered that any variations and/or modifications may be made by experts in the field without departing from the relative scope of protection, as defined by the appended claims.

### References

1.Havel, J. J. The evolving landscape of biomarkers for checkpoint inhibitor immunotherapy. Nat. Rev. Cancer doi:10.1038/s41568-019-0116-x
2.Aurisicchio, L., Pallocca, M., Ciliberto, G. & Palombo, F. The perfect personalized cancer therapy: cancer vaccines against neoantigens. J. Exp. Clin. Cancer Res. 37, 86 (2018).
3.Carreno, B. M. et al. A dendritic cell vaccine increases the breadth and diversity of melanoma neoantigen-specific T cells. Science (80-. ). 348, 803-808 (2015).
4.Ott, P. A. et al. An immunogenic personal neoantigen vaccine for patients with melanoma. Nature 547, 217-221 (2017).
5.Sahin, U. et al. Personalized RNA mutanome vaccines mobilize poly-specific therapeutic immunity against cancer. Nature 547, 222-226 (2017).
6.Keskin, D. B. et al. Neoantigen vaccine generates intratumoral T cell responses in phase Ib glioblastoma trial. (2018). doi:10.1038/s41586-018-0792-9
7.Hilf, N. et al. Actively personalized vaccination trial for newly diagnosed glioblastoma. Nature 565, 240-245 (2019).
8.Alise, A. M. D. et al. as strategy to eradicate large tumors combined. Nat. Commun. 1-12 doi:10.1038/s41467-019-10594-2
9.Aurisicchio, L. et al. Poly-specific neoantigen-targeted cancer vaccines delay patient derived tumor growth. 4, 1-13 (2019).
10.Andre, F. & Mir, L. M. DNA electrotransfer: its principles and an updated review of its therapeutic applications. Gene Ther. 11 Suppl 1, S33-42 (2004).
11.Gothelf, A. & Gehl, J. What you always needed to know about electroporation based DNA vaccines. Hum. Vaccin. Immunother. 8, 1694-1702 (2012).
12.Neumann, E., Kakorin, S. & Toensing, K. Fundamentals of electroporative delivery of drugs and genes. Bioelectrochem. Bioenerg. 48, 3-16 (1999).
13.Lurquin, P. F. Gene transfer by electroporation. Mol. Biotechnol. 7, 5-35 (1997).
14.Mathiesen, I. Electropermeabilization of skeletal muscle enhances gene transfer in vivo. Gene Ther. 6, 508-514 (1999).
15.Mir, L. M. et al. High-efficiency gene transfer into skeletal muscle mediated by electric pulses. Proc. Natl. Acad. Sci. U. S. A. 96, 4262-4267 (1999).
16.Rizzuto, G. et al. Gene electrotransfer results in a high-level transduction of rat skeletal muscle and corrects anemia of renal failure. Hum. Gene Ther. 11, 1891-1900 (2000).
17.Fattori, E., the Monica, N., Ciliberto, G. & Toniatti, C. Electrogene-transfer: a new approach for muscle gene delivery. Somat. Cell Mol. Genet. 27, 75-83 (2002).
18.Durieux, A.-C., Bonnefoy, R., Busso, T. & Freyssenet, D. In vivo gene electrotransfer into skeletal muscle: effects of plasmid DNA on the occurrence and extent of muscle damage. J. Gene Med. 6, 809-816 (2004).
19.Diaz, C. M. et al. Phase 1 studies of the safety and immunogenicity of electroporated HER2/CEA DNA vaccine followed by adenoviral boost immunization in patients with solid tumors. J. Transl. Med. 11, 62 (2013).
20.Nielsen, M. et al. Reliable prediction of T-cell epitopes using neural networks with novel sequence representations. Protein Sci. 12, 1007-1017 (2003).
21. Kreiter, M. et al, Mutant MHC class II epitopes drive therapeutic immune responses to cancer. Nature 20(7549): 692-696, doi:10.1038/nature14426 (2015)
22. Yadav 2014 doi: 10.1038/nature14001

## Claims

1. Combination of a first component A comprising:
a modulator of the immune response, preferably an anti-CTLA-4 antibody, or
a polynucleotide encoding said modulator, or
an expression vector or plasmid which comprises the polynucleotide encoding said modulator with a second component B comprising:
a polynucleotide encoding one or more neoantigens, or
an expression vector or plasmid comprising said polynucleotide encoding one or more neoantigens,
said combination being for simultaneous, separate or sequential use in the prevention and treatment of tumors.

2. Combination according to claim 1, for use according to claim 1, wherein said modulator of the immune response is selected in the group consisting of anti- CD27, CD40, OX40, GITR, PD1, PD-L1, CD274, A2AR, B7-H3, B7-H4, BTLA, CTLA-4, IDO, KIR, LAG3, TIM-3, VISTA, IL-2, IL-12, IL-15 antibodies.

3. Combination according to any one of claims 1-2, for use according to claim 1, wherein said polynucleotide encoding said modulator and/or said one or more neoantigens or expression vector or plasmid comprising said polynucleotide is administered by electroporation.

4. Combination according to any one of claims 1-3, for use according to claim 1, wherein said polynucleotide encoding one or more neoantigens comprises, at the 5' end, a leader sequence, for example TPA.

5. Combination according to any one of claims 1-4, for use according to claim 1, wherein said one or more neoantigens are at least 3 neoantigens, preferably from 3 to 50 neoantigens, more preferably at least 3, 5, 8, 10, 20, 30, 40, for example 3, 5, 8, 10, 20, 30, 40, or 50 neoantigens, even more preferably at least 20, alone or in combination with tumor antigenic epitopes.

6. Combination according to any one of claims 1-5, for use according to claim 1, which further comprises an immune response promoter, for example a probiotic, and/or an antineoplastic agent.

7. Combination according to any one of claims 1-6, for use according to claim 1, wherein the tumor is selected in the group consisting of melanoma, lung cancer, tumors of the urinary system, such as, for example, of the kidney, urethra or bladder, tumor of the digestive system, such as, for example, of the esophagus, stomach, intestine, colon, colon/rectum, anus, liver, gall bladder, biliary ducts or pancreas, breast cancer, eye tumor, brain tumor, lymphomas, such as, for example, Hodgkin and non-Hodgkin, multiple myeloma, leukemias, such as, for example, myeloid and lymphocytic chronic leukemia, and/or tumor of the reproductive system, such as, for example, of the cervix, endometrium, ovary, prostate, testicles, penis or vulva.

8. Kit for the prevention and treatment of tumors, said kit comprising:
a) a first component A comprising:
a modulator of the immune response, preferably an anti-CTLA-4 antibody, or
a polynucleotide encoding said modulator, or
an expression vector or plasmid which comprises the polynucleotide encoding said modulator;
b) a second component B comprising:
a polynucleotide encoding one or more neoantigens, or
an expression vector or plasmid comprising said polynucleotide encoding one or more neoantigens;
c) a system of administration by electroporation.
